# EUROPEAN PATENT APPLICATION

(11) **EP 2 048 232 A1**
(43) Date of publication of application: **15.04.2009**
(21) Application number: 07768079.1
(22) Date of filing: 03.07.2007
(51) Int. Cl.: C12N 15/09, A61K 48/00, C12N 7/00, C12N 5/10

(54) **NON-REPLICATING PARAMYXOVIRIDAE VIRUS VECTOR**

(30) Priority: 13.07.2006 JP 2006193433
(71) Applicant: Dnavec Corporation, Tsukuba-shi, Ibaraki 305-0856 (JP)
(72) Inventor: YOU, Jun, Tsukuba-shi Ibaraki 305-0856 (JP); INOUE, Makoto, Tsukuba-shi Ibaraki 305-0856 (JP); HASEGAWA, Mamoru, Tsukuba-shi Ibaraki 305-0856 (JP)
(74) Representative: Vossius & Partner
(86) International application number: PCT/JP2007/063305
(87) International publication number: WO 2008/007581

(57) **Abstract**

The present inventors succeeded in producing non-replicating SeV vectors whose genomic RNAs lack all genes for the NP, P, and L proteins, which are RNP-constituting proteins. The present inventors confirmed that the NP/P/L-deficient SeV vectors carrying a marker gene such as GFP provide high productivity, and high transfer and expression efficiencies of foreign genes (high MOI infection is essential for achieving high expression levels). By lacking the L gene or two or more of the NP, P, and L genes, the vectors of the present invention enable lowering the level of virus-derived proteins expressed in host cells, thereby reducing the immunogenicity upon *in vivo* administration.

SEQUENCE LISTING

<110> DNAVEC CORPORATION

<120> Non-replicating paramyxoviridae virus vector

<130> D4-A0602P

<140> PCT/JP2007/063305
<141> 2007-07-03

<150> JP 2006193433
<151> 2006-07-13

<160> 21

<170> PatentIn version 3.4

<210> 1
<211> 33
<212> DNA
<213> Artificial

<220>
<223> An artificially synthesized primer sequence

<400> 1
aagtggcggc cgcacggcgc aatggcagat atc 33

<210> 2
<211> 70
<212> DNA
<213> Artificial

<220>

<223>  An artificially synthesized primer sequence

<400>  2
ggtaccgcgg agcttcgatc gttctgcacg atagggacta attattaaga ctcggccttg      60

cataatttag                                                              70

<210>  3
<211>  5028
<212>  DNA
<213>  Artificial

<220>
<223>  An artificially synthesized vector sequence

<400>  3
accaaacaag agaaaaaaca tgtatgggat atgtaatgaa gttatacagg attttagggt      60

caaagtatcc accctgagga gcaggttcca gaccctttgc tttgctgcca aagttcacgc     120

ggccgcacgg cgcaatggca gatatctata gattccctaa gttctcatat gaggataacg     180

gtactgtgga gcccctgcct ctgagaactg gtccggataa gaaagccatc ccccacatca     240

ggattgtcaa ggtaggagac cctcctaaac atggagtgag atacctagat ttattgctct     300

tgggtttctt tgagacaccg aaacaaacaa ccaatctagg gagcgtatct gacttgacag     360

agccgaccag ctactcaata tgcggctccg ggtcgttacc cataggtgtg gccaaatact     420

acgggactga tcaggaactc ttaaaggcct gcaccgatct cagaattacg gtgaggagga     480

ctgttcgagc aggagagatg atcgtataca tggtggattc gattggtgct ccactcctac     540

catggtcagg caggctgaga cagggaatga tatttaatgc aaacaaggtc gcactagctc     600

cccaatgcct ccctgtggac aaggacataa gactcagagt ggtgtttgtc aatgggacat 660

ctctaggggc aatcaccata gccaagatcc caaagaccct tgcagacctt gcattgccca 720

actctatatc tgttaattta ctggtgacac tcaagaccgg gatctccaca gaacaaaagg 780

gggtactccc agtacttgat gatcaagggg agaaaaagct caattttatg gtgcacctcg 840

ggttgatcag gagaaaggtc gggaagatat actctgttga gtactgcaag agcaagattg 900

agagaatgcg gctgattttc tcacttgggt taatcggcgg tataagcttc catgttcagg 960

ttaatgggac actatctaag acattcatga gtcagctcgc atggaagagg gcagtctgct 1020

tcccattaat ggatgtgaat ccccatatga acatggtgat ttgggcggca tctgtagaaa 1080

tcacaggcgt cgatgcggtg ttccaaccgg ccatccctcg tgatttccgc tactacccta 1140

atgttgtggc taagaacatc ggaaggatca gaaagctgta aatgtgcacc catcagagac 1200

ctgcgacaat gccccaagca gacaccacct ggcagtcgga gccaccgggt cactccttgt 1260

cttaaataag aaaaacttag ggataaagtc ccttgtgagt gcttggttgc aaaactctcc 1320

ccttgggaaa catgacagca tatatccaga gatcacagtg catctcaaca tcactactgg 1380

ttgttctcac cacattggtc tcgtgtcaga ttcccaggga taggctctct aacatagggg 1440

tcatagtcga tgaagggaaa tcactgaaga tagctggatc ccacgaatcg aggtacatag 1500

tactgagtct agttccgggg gtagactttg agaatgggtg cggaacagcc caggttatcc 1560

agtacaagag cctactgaac aggctgttaa tcccattgag ggatgcctta gatcttcagg 1620

aggctctgat aactgtcacc aatgatacga cacaaaatgc cggtgctccc cagtcgagat 1680

tcttcggtgc tgtgattggt actatcgcac ttggagtggc gacatcagca caaatcaccg 1740

cagggattgc actagccgaa gcgagggagg ccaaaagaga catagcgctc atcaaagaat 1800

cgatgacaaa aacacacaag tctatagaac tgctgcaaaa cgctgtgggg gaacaaattc 1860

ttgctctaaa gacactccag gatttcgtga atgatgagat caaacccgca ataagcgaat 1920

taggctgtga gactgctgcc ttaagactgg gtataaaatt gacacagcat tactccgagc 1980

tgttaactgc gttcggctcg aatttcggaa ccatcggaga gaagagcctc acgctgcagg 2040

cgctgtcttc actttactct gctaacatta ctgagattat gaccacaatc aggacagggc 2100

agtctaacat ctatgatgtc atttatacag aacagatcaa aggaacggtg atagatgtgg 2160

atctagagag atacatggtc accctgtctg tgaagatccc tattctttct gaagtcccag 2220

gtgtgctcat acacaaggca tcatctattt cttacaacat agacggggag gaatggtatg 2280

tgactgtccc cagccatata ctcagtcgtg cttctttctt agggggtgca gacataaccg 2340

attgtgttga gtccagattg acctatatat gccccaggga tcccgcacaa ctgatacctg 2400

acagccagca aaagtgtatc ctgggggaca caacaaggtg tcctgtcaca aaagttgtgg 2460

acagccttat ccccaagttt gcttttgtga atgggggcgt tgttgctaac tgcatagcat 2520

ccacatgtac ctgcgggaca ggccgaagac caatcagtca ggatcgctct aaaggtgtag 2580

tattcctaac ccatgacaac tgtggtctta taggtgtcaa tggggtagaa ttgtatgcta 2640

accggagagg gcacgatgcc acttgggggg tccagaactt gacagtcggt cctgcaattg 2700

ctatcagacc cgttgatatt tctctcaacc ttgctgatgc tacgaatttc ttgcaagact 2760

ctaaggctga gcttgagaaa gcacggaaaa tcctctcgga ggtaggtaga tggtacaact 2820

caagagagac tgtgattacg atcatagtag ttatggtcgt aatattggtg gtcattatag 2880

tgatcatcat cgtgctttat agactcagaa ggtcaatgct aatgggtaat ccagatgacc 2940

gtataccgag ggacacatac acattagagc cgaagatcag acatatgtac acaaacggtg 3000

ggtttgatgc aatggctgag aaaagatgat cacgaccatt atcagatgtc ttgtaaagca 3060

ggcatagtat ccgttgagat ctgtatataa taagaaaaac ttagggtgaa agtgaggtcg 3120

cgcggtactt tagctttcac ctcaaacaag cacagatcat ggatggtgat aggggcaaac 3180

gtgactcgta ctggtctact tctcctagtg gtagcaccac aaaaccagca tcaggttggg 3240

agaggtcaag taaagccgac acatggttgc tgattctctc attcacccag tgggctttgt 3300

caattgccac agtgatcatc tgtatcataa tttctgctag acaagggtat agtatgaaag 3360

agtactcaat gactgtagag gcattgaaca tgagcagcag ggaggtgaaa gagtcactta 3420

ccagtctaat aaggcaagag gttatagcaa gggctgtcaa cattcagagc tctgtgcaaa 3480

ccggaatccc agtcttgttg aacaaaaaca gcagggatgt catccagatg attgataagt 3540

cgtgcagcag acaagagctc actcagcact gtgagagtac gatcgcagtc caccatgccg 3600

atggaattgc cccacttgag ccacatagtt tctggagatg ccctgtcgga gaaccgtatc 3660

ttagctcaga tcctgaaatc tcattgctgc ctggtccgag cttgttatct ggttctacaa 3720

cgatctctgg atgtgttagg ctcccttcac tctcaattgg cgaggcaatc tatgcctatt 3780

catcaaatct cattacacaa ggttgtgctg acatagggaa atcatatcag gtcctgcagc 3840

tagggtacat atcactcaat tcagatatgt tccctgatct taaccccgta gtgtcccaca 3900

cttatgacat caacgacaat cggaaatcat gctctgtggt ggcaaccggg actaggggtt 3960

atcagctttg ctccatgccg actgtagacg aaagaaccga ctactctagt gatggtattg 4020

aggatctggt ccttgatgtc ctggatctca aagggagaac taagtctcac cggtatcgca 4080

acagcgaggt agatcttgat cacccgttct ctgcactata ccccagtgta ggcaacggca 4140

ttgcaacaga aggctcattg atatttcttg ggtatggtgg actaaccacc cctctgcagg 4200

gtgatacaaa atgtaggacc caaggatgcc aacaggtgtc gcaagacaca tgcaatgagg 4260

ctctgaaaat tacatggcta ggagggaaac aggtggtcag cgtgatcatc caggtcaatg 4320

actatctctc agagaggcca aagataagag tcacaaccat tccaatcact caaaactatc 4380

tcggggcgga aggtagatta ttaaaattgg gtgatcgggt gtacatctat acaagatcat 4440

caggctggca ctctcaactg cagataggag tacttgatgt cagccaccct ttgactatca 4500

actggacacc tcatgaagcc ttgtctagac caggaaataa agagtgcaat tggtacaata 4560

agtgtccgaa ggaatgcata tcaggcgtat acactgatgc ttatccattg tcccctgatg 4620

cagctaacgt cgctaccgtc acgctatatg ccaatacatc gcgtgtcaac ccaacaatca 4680

tgtattctaa cactactaac attataaata tgttaaggat aaaggatgtt caattagagg 4740

ctgcatatac cacgacatcg tgtatcacgc attttggtaa aggctactgc tttcacatca 4800

tcgagatcaa tcagaagagc ctgaatacct tacagccgat gctctttaag actagcatcc 4860

ctaaattatg caaggccgag tcttaataat tagtccctat cgtgcagaac gatcgaagct 4920

ccgcggtacc tggaagtctt ggacttgtcc atatgacaat agtaagaaaa acttacaaga 4980

agacaagaaa atttaaaagg atacatatct cttaaactct tgtctggt 5028

<210> 4
<211> 788
<212> DNA
<213> Artificial

<220>
<223> An artificially synthesized nucleotide sequence

<400> 4
gcggccgccg tacggccatg gtgagcaagg gcgaggagct gttcaccggg gtggtgccca 60

tcctggtcga gctggacggc gacgtaaacg gccacaagtt cagcgtgtcc ggcgagggcg 120

agggcgatgc cacctacggc aagctgaccc tgaagttcat ctgcaccacc ggcaagctgc 180

ccgtgccctg gcccaccctc gtgaccaccc tgacctacgg cgtgcagtgc ttcagccgct 240

accccgacca catgaagcag cacgacttct tcaagtccgc catgcccgaa ggctacgtcc 300

aggagcgcac catcttcttc aaggacgacg gcaactacaa gacccgcgcc gaggtgaagt 360

tcgagggcga caccctggtg aaccgcatcg agctgaaggg catcgacttc aaggaggacg 420

gcaacatcct ggggcacaag ctggagtaca actacaacag ccacaacgtc tatatcatgg 480

ccgacaagca gaagaacggc atcaaggtga acttcaagat ccgccacaac atcgaggacg 540

gcagcgtgca gctcgccgac cactaccagc agaacacccc catcggcgac ggccccgtgc 600

tgctgcccga caaccactac ctgagcaccc agtccgccct gagcaaagac cccaacgaga 660

agcgcgatca catggtcctg ctggagttcg tgaccgccgc cgggatcact ctcggcatgg 720

acgagctgta caagtaaagc tccgaagtaa gaaaaactta gggtgaaagt tcatcgtacg 780

gcggccgc 788

<210> 5
<211> 55
<212> DNA
<213> Artificial

<220>
<223> An artificially synthesized primer sequence

<400> 5
acttgaattc acggcttcgg ctacacttac cgcctggatc aagatgcctt cattc 55

<210> 6
<211> 30
<212> DNA
<213> Artificial

<220>
<223> An artificially synthesized primer sequence

<400> 6
cgaggcggcc gcttactctt gcactatgtg 30

<210> 7
<211> 662
<212> DNA
<213> Artificial

<220>
<223>  An artificially synthesized nucleotide sequence

<400>  7
gaattcacgg cttcggctac acttaccgcc tggatcaaga tgccttcatt cttaaagaag      60

attctgaagt tgagagggag gcgccaggag gacgagagtc gctctcggat gttatcggat     120

tcctcgatgc tgtcctgtcg agtgaaccaa ctgacatcgg aggggacaga agctggctcc     180

acaacaccat caacactccc caaggaccag gctctgctca tagagccaaa agtgagggcg     240

aaggagaagt ctcaacaccg tcgacccaag ataatcgatc aggtgaggag agtagagtct     300

ctgggagaac aagcaagcca gaggcagaag cacatgctgg aaaccttgat aaacaaaata     360

tacaccgggc ctttggggga agaactggta caaactctgt atctcaggat ctgggcgatg     420

gaggagactc cggaatcctt gaaaatcctc caaatgagag aggatatccg agatcaggta     480

ttgaagatga aaacagagag atggctgcgc accctgataa gaggggagaa gaccaagctg     540

aaggacttcc agaagaggta cgaggaagta catccctacc tgatgaagga gaaggtggag     600

caagtaataa tggaagaagc atggagcctg gcagctcaca tagtgcaaga gtaagcggcc     660

gc                                                                    662

<210>  8
<211>  10
<212>  DNA
<213>  Sendai virus

<400>  8

ctttcaccct                                                          10

<210>  9
<211>  15
<212>  DNA
<213>  Sendai virus

<400>  9
tttttcttac tacgg                                                    15

<210>  10
<211>  50
<212>  DNA
<213>  Artificial

<220>
<223>  An artificially synthesized primer sequence

<400>  10
agttcacgcg gccgcagatc ttcacgatgg tgagcaaggg cgaggagctg              50

<210>  11
<211>  65
<212>  DNA
<213>  Artificial

<220>
<223>  An artificially synthesized primer sequence

<400>  11
caggtaccgc ggagcttcga tcgttctgca cgatagggac taattacttg tacagctcgt      60

ccatg                                                               65

<210> 12
<211> 996
<212> DNA
<213> Artificial

<220>
<223> An artificially synthesized vector sequence

<220>
<221> misc_feature
<222> (138)..(857)
<223> Open reading frame sequence of GFP

<400> 12
accaaacaag agaaaaaaca tgtatgggat atgtaatgaa gttatacagg attttagggt 60

caaagtatcc accctgagga gcaggttcca gaccctttgc tttgctgcca aagttcacgc 120

ggccgcagat cttcacgatg gtgagcaagg gcgaggagct gttcaccggg gtggtgccca 180

tcctggtcga gctggacggc gacgtaaacg gccacaagtt cagcgtgtcc ggcgagggcg 240

agggcgatgc cacctacggc aagctgaccc tgaagttcat ctgcaccacc ggcaagctgc 300

ccgtgccctg gcccaccctc gtgaccaccc tgacctacgg cgtgcagtgc ttcagccgct 360

accccgacca catgaagcag cacgacttct tcaagtccgc catgcccgaa ggctacgtcc 420

aggagcgcac catcttcttc aaggacgacg gcaactacaa gacccgcgcc gaggtgaagt 480

tcgagggcga caccctggtg aaccgcatcg agctgaaggg catcgacttc aaggaggacg 540

gcaacatcct ggggcacaag ctggagtaca actacaacag ccacaacgtc tatatcatgg 600

ccgacaagca gaagaacggc atcaaggtga acttcaagat ccgccacaac atcgaggacg 660

gcagcgtgca gctcgccgac cactaccagc agaacacccc catcggcgac ggccccgtgc 720

tgctgcccga caaccactac ctgagcaccc agtccgccct gagcaaagac cccaacgaga 780

agcgcgatca catggtcctg ctggagttcg tgaccgccgc cgggatcact ctcggcatgg 840

acgagctgta caagtaatta gtccctatcg tgcagaacga tcgaagctcc gcggtacctg 900

gaagtcttgg acttgtccat atgacaatag taagaaaaac ttacaagaag acaagaaaat 960

ttaaaaggat acatatctct taaactcttg tctggt 996

<210> 13
<211> 50
<212> DNA
<213> Artificial

<220>
<223> An artificially synthesized primer sequence

<400> 13
atccatcgcg gccgcagatc ttcacgatgg aagacgccaa aaacataaag 50

<210> 14
<211> 66
<212> DNA
<213> Artificial

<220>
<223> An artificially synthesized primer sequence

<400>  14
acttactccg cggttcgatc gttctgcacg atagggacta attacacggc gatctttccg      60

cccttc                                                                  66

<210>  15
<211>  1706
<212>  DNA
<213>  Artificial

<220>
<223>  An artificially synthesized vector sequence

<220>
<221>  misc_feature
<222>  (20)..(1672)
<223>  Open reading frame sequence of Luciferase

<400>  15
gcggccgcag atcttcacga tggaagacgc caaaaacata aagaaaggcc cggcgccatt      60

ctatccgctg gaagatggaa ccgctggaga gcaactgcat aaggctatga agagatacgc     120

cctggttcct ggaacaattg cttttacaga tgcacatatc gaggtggaca tcacttacgc     180

tgagtacttc gaaatgtccg ttcggttggc agaagctatg aaacgatatg ggctgaatac     240

aaatcacaga atcgtcgtat gcagtgaaaa ctctcttcaa ttctttatgc cggtgttggg     300

cgcgttattt atcggagttg cagttgcgcc cgcgaacgac atttataatg aacgtgaatt     360

gctcaacagt atgggcattt cgcagcctac cgtggtgttc gtttccaaaa aggggttgca     420

aaaaattttg aacgtgcaaa aaaagctccc aatcatccaa aaaattatta tcatggattc     480

taaaacggat taccagggat ttcagtcgat gtacacgttc gtcacatctc atctacctcc 540

cggttttaat gaatacgatt ttgtgccaga gtccttcgat agggacaaga caattgcact 600

gatcatgaac tcctctggat ctactggtct gcctaaaggt gtcgctctgc ctcatagaac 660

tgcctgcgtg agattctcgc atgccagaga tcctattttt ggcaatcaaa tcattccgga 720

tactgcgatt ttaagtgttg ttccattcca tcacggtttt ggaatgttta ctacactcgg 780

atatttgata tgtggatttc gagtcgtctt aatgtataga tttgaagaag agctgtttct 840

gaggagcctt caggattaca agattcaaag tgcgctgctg gtgccaaccc tattctcctt 900

cttcgccaaa agcactctga ttgacaaata cgatttatct aatttacacg aaattgcttc 960

tggtggcgct cccctctcta aggaagtcgg ggaagcggtt gccaagaggt tccatctgcc 1020

aggtatcagg caaggatatg ggctcactga gactacatca gctattctga ttacacccga 1080

gggggatgat aaaccgggcg cggtcggtaa agttgttcca ttttttgaag cgaaggttgt 1140

ggatctggat accgggaaaa cgctgggcgt taatcaaaga ggcgaactgt gtgtgagagg 1200

tcctatgatt atgtccggtt atgtaaacaa tccggaagcg accaacgcct tgattgacaa 1260

ggatggatgg ctacattctg gagacatagc ttactgggac gaagacgaac acttcttcat 1320

cgttgaccgc ctgaagtctc tgattaagta caaaggctat caggtggctc ccgctgaatt 1380

ggaatccatc ttgctccaac accccaacat cttcgacgca ggtgtcgcag gtcttcccga 1440

cgatgacgcc ggtgaacttc ccgccgccgt tgttgttttg gagcacggaa agacgatgac 1500

ggaaaaagag atcgtggatt acgtcgccag tcaagtaaca accgcgaaaa agttgcgcgg 1560

aggagttgtg tttgtggacg aagtaccgaa aggtcttacc ggaaaactcg acgcaagaaa 1620

aatcagagag atcctcataa aggccaagaa gggcggaaag atcgccgtgt aattagtccc 1680

tatcgtgcag aacgatcgaa ccgcgg 1706

<210> 16
<211> 22
<212> DNA
<213> Artificial

<220>
<223> An artificially synthesized primer sequence

<400> 16
ataccgcatc aggcgccatt cg 22

<210> 17
<211> 57
<212> DNA
<213> Artificial

<220>
<223> An artificially synthesized primer sequence

<400> 17
   gttttttagg gtctggaacc tgctcctcag ggtggatact ttgacactaa aatcctg 57

<210> 18
<211> 34
<212> DNA
<213> Artificial

<220>

<223>  An artificially synthesized primer sequence

<400>  18
ggttccagac cctaaaaaac atgtatggga tatg                                34

<210>  19
<211>  1080
<212>  DNA
<213>  Artificial

<220>
<223>  An artificially synthesized vector sequence

<220>
<221>  misc_feature
<222>  (222)..(941)
<223>  Open reading frame sequence of GFP

<400>  19
accaaacaag agaaaaaaca tgtatgggat atgtaatgaa gttatacagg attttagtgt      60

caaagtatcc accctgagga gcaggttcca gaccctaaaa aacatgtatg ggatatgtaa     120

tgaagttata caggatttta gggtcaaagt atccaccctg aggagcaggt tccagaccct     180

ttgctttgct gccaaagttc acgcggccgc agatcttcac gatggtgagc aagggcgagg     240

agctgttcac cggggtggtg cccatcctgg tcgagctgga cggcgacgta aacggccaca     300

agttcagcgt gtccggcgag ggcgagggcg atgccaccta cggcaagctg accctgaagt     360

tcatctgcac caccggcaag ctgcccgtgc cctggcccac cctcgtgacc accctgacct     420

acggcgtgca gtgcttcagc cgctaccccg accacatgaa gcagcacgac ttcttcaagt 480

ccgccatgcc cgaaggctac gtccaggagc gcaccatctt cttcaaggac gacggcaact 540

acaagacccg cgccgaggtg aagttcgagg gcgacaccct ggtgaaccgc atcgagctga 600

agggcatcga cttcaaggag gacggcaaca tcctggggca caagctggag tacaactaca 660

acagccacaa cgtctatatc atggccgaca agcagaagaa cggcatcaag gtgaacttca 720

agatccgcca caacatcgag gacggcagcg tgcagctcgc cgaccactac cagcagaaca 780

cccccatcgg cgacggcccc gtgctgctgc ccgacaacca ctacctgagc acccagtccg 840

ccctgagcaa agaccccaac gagaagcgcg atcacatggt cctgctggag ttcgtgaccg 900

ccgccgggat cactctcggc atggacgagc tgtacaagta attagtccct atcgtgcaga 960

acgatcgaag ctccgcggta cctggaagtc ttggacttgt ccatatgaca atagtaagaa 1020

aaacttacaa gaagacaaga aaatttaaaa ggatacatat ctcttaaact cttgtctggt 1080

<210> 20
<211> 96
<212> DNA
<213> Artificial

<220>
<223> An artificially synthesized promoter sequence

<400> 20
accaaacaag agaaaaaaca tgtatgggat atgtaatgaa gttatacagg attttagggt 60

caaagtatcc accctgagga gcaggttcca gaccct 96

<210>  21

<211>  180

<212>  DNA

<213>  Artificial

<220>

<223>  An artificially synthesized promoter sequence

<400>  21

accaaacaag agaaaaaaca tgtatgggat atgtaatgaa gttatacagg attttagtgt      60

caaagtatcc accctgagga gcaggttcca gaccctaaaa aacatgtatg ggatatgtaa     120

tgaagttata caggatttta gggtcaaagt atccaccctg aggagcaggt tccagaccct     180

## Description

### Technical Field

The present invention relates to non-replicating *Paramyxoviridae* virus vectors.

### Background Art

Sendai virus vectors (SeV vector) are cytoplasmic viral vectors, and their expression all phases are all carried out within the cytoplasm. Thus, even when the vectors are used *in vivo*, there is no worry that a carried gene will become integrated into the host chromosome and cause genetic toxicity. Furthermore, these vectors have a number of excellent characteristics, such as high gene transfer and expression efficiencies both *in vitro* and *in vivo*, and long-term sustained expression *in vitro*. Thus, SeV is expected to have many applications and uses as a gene transfer vector in gene therapy, gene vaccination, antibody production, functional analysis and such (Non-Patent Documents 1 and 2).

However, there is a possibility that immunogenicity may be induced when Sendai virus vectors are used to express foreign genes in *in vivo* applications, because these vectors express foreign genes that they carry as well as the viral structural proteins (in particular, the NP, P, and L proteins, which are RNP-constituting proteins essential for transcription and replication). In recent years, various types of gene-deficient SeV vectors have been developed, and they include, for example, vectors that are deficient in one or more of the M, F, and HN proteins, which are not viral RNP-constituting proteins (Patent Documents 1 and 2). Although reduced immunogenicity is still present in these gene-deficient SeV vectors.

NP, P, and L, which are proteins constituting the SeV vector RNP, are necessary for transcription and replication of the Sendai virus genome, and thus are normally essential for the SeV vector. However, even when the genome is deficient in these protein genes, theoretically viral vector particles can be reconstituted and amplified by externally supplying these proteins from expression plasmids or such. Furthermore, when target cells are infected with reconstituted SeV vectors deficient in NP, P, and L, a foreign gene in the genome can be expressed by transcribing the gene's mRNA using the NP, P, and L proteins carried in the particles. The SeV vectors deficient in NP, P, and L cannot replicate RNP due to deficiency of the NP, P, and L genes in the genome, and thus can be turned into non-replicating SeV vectors.

When the genome is deficient in any one ofNP, P, and L, replication of RNP becomes impossible (or is extremely reduced), and thus the vector can serve as a non-replicating SeV vector. It was previously reported that vectors deficient in either of the NP or P protein gene were constructed to produce non-replicating SeV vectors (Non-Patent Documents 3 and 4). However, in consideration of the fact that the L protein itself has RNA polymerase activity, the characteristics of a non-replicating vector can be assured by deleting the L gene or multiple genes including the L gene, rather than by deleting only the P or NP gene from the genome. In addition, the influences of virus-derived proteins are expected to be minimized by deleting multiple genes comprising the L gene. Thus, deletion of multiple genes including the L gene, for example, all of the NP, P, and L genes, is preferable from the viewpoint of immunogenicity reduction. Deletion of the L gene, which occupies about half of the genome length, is expected to enable the genome to carry a larger gene of interest. Moreover, the smaller the genome size is, the higher the transcription efficiency is, and the higher the expression level of the gene of interest is expected to be. Thus, L gene-deficient SeV vectors are considered to be very useful. However, there has been no report on the production of L gene-deficient vectors for *Paramyxoviridae* virus vectors, including SeV vectors.
[Patent Document 1]
   WO00/70070
[Patent Document 2]
   WO2003/025570
[Non-Patent Document 1]
   Bitzer M, et al., J Gene Med. 5(7):543-553 (2003)
[Non-Patent Document 2]
   Griesenbach U, et al., Curr Opin Mol Ther. 7(4):346-352. (2005)
[Non-Patent Document 3]
   Molecular Therapy Volume 13, Supplement 1, May 2006, page S185
[Non-Patent Document 4]
   Abstracts of NSV2006 (13th International Conference Negative Strand Viruses 2006, Salamance, Spain, June 17-22nd, 2006)

### Disclosure of the Invention

### [Problems to be Solved by the Invention]

The present invention was achieved in view of the circumstances described above. An objective of the present invention is to produce non-replicating *Paramyxoviridae* virus vectors with low immunogenicity.

### [Means for Solving the Problems]

The present inventors conducted dedicated studies to achieve the objective described above. The present inventors successfully produced non-replicating SeV vectors that lack in their genomic RNAs genes for the NP, P, and L proteins, which are RNP-constituting proteins. The present inventors confirmed that the NP/P/L-deficient SeV vectors carrying a marker gene such as GFP provided high productivity (1e7 CIU/ml or more), and high transfer and expression efficiencies of foreign genes (high MOI infection is essential for achieving high expression levels). Vectors of the present invention are deficient in the L gene or more than one of the NP, P, and L genes, and can therefore lower the level of virus-derived proteins expressed in host cells, reducing the immunogenicity upon *in vivo* administration. In particular, the vectors of the present invention are very useful in the field of gene therapy. Specifically, the present invention relates to L gene-deficient *Paramyxoviridae* virus vectors. More specifically, the present invention provides the following:
(1) a vector that comprises a complex comprising:
   (a) a negative single-stranded RNA derived from a *Paramyxoviridae* virus, wherein said negative single-stranded RNA has been modified so as not to express one or more proteins, selected from the NP, P, and L proteins, including at least the L protein, which bind to a *Paramyxoviridae* virus negative single-stranded RNA; and
   (b) the NP, P, and L proteins;
(2) a vector that comprises a complex comprising:
   (a) a negative single-stranded RNA derived from a *Paramyxoviridae* virus, wherein said negative single-stranded RNA has been modified so as not to express multiple proteins selected from the NP, P, and L proteins, which bind to a *Paramyxoviridae* virus negative single-stranded RNA; and
   (b) the NP, P, and L proteins;
(3) the vector of (1) or (2), wherein the *Paramyxoviridae* virus-derived negative single-stranded RNA has been modified so as not to express the NP, P, and L proteins;
(4) the vector of any one of (1) to (3), wherein the *Paramyxoviridae* virus-derived negative single-stranded RNA expresses at least one of the *Paramyxoviridae* virus envelope proteins;
(5) the vector of (4), wherein the *Paramyxoviridae* virus-derived negative single-stranded RNA has been modified so as to express the M, F, and HN proteins, and not to express the NP, P, and L proteins;
(6) the vector of any one of (1) to (5), wherein the negative single-stranded RNA is derived from Sendai virus;
(7) the vector of any one of (1) to (6), wherein the negative single-stranded RNA further carries a foreign gene;
(8) a DNA encoding a negative single-stranded RNA comprised in the vector of any one of (1) to (7), or the complementary strand thereof;
(9) a method for producing the vector of (1), wherein the method comprises the steps of:
   (a) introducing a DNA encoding a *Paramyxoviridae* virus-derived negative single-stranded RNA, or the complementary strand thereof, into cells capable of expressing the NP, P, and L proteins,
      wherein said negative single-stranded RNA has been modified so as not to express one or more proteins, including at least the L protein selected from the NP, P, and L proteins, which bind to a *Paramyxoviridae* virus negative single-stranded RNA; and
   (b) culturing the cells, and collecting viral particles from the culture supernatant;
(10) a method for producing the vector of (2), wherein the method comprises the steps of:
   (a) introducing a DNA encoding a *Paramyxoviridae* virus-derived negative single-stranded RNA, or the complementary strand thereof, into cells capable of expressing the NP, P, and L proteins,
      wherein said negative single-stranded RNA has been modified so as not to express multiple proteins selected from the NP, P, and L proteins; and
   (b) culturing the cells, and collecting viral particles from the culture supernatant;
(11) the production method of (9) or (10), wherein the cells capable of expressing the NP, P, and L proteins can further express a protein having the function of promoting formation and release of viral particles;
(12) the method of (11), wherein the protein having the function of promoting formation and release of viral particles is the C protein;
(13) a method for producing the vector of (1), wherein the method comprises the steps of:
   (a) introducing a vector expressing the NP protein, a vector expressing the P protein, a vector expressing the L protein, and a vector into which a DNA encoding a *Paramyxoviridae* virus-derived negative single-stranded RNA, or the complementary strand thereof, is inserted, into cells capable of expressing the vectors,
      wherein said negative single-stranded RNA has been modified so as not to express one or more proteins, including at least the L protein selected from the NP, P, and L proteins, which bind to a *Paramyxoviridae* virus negative single-stranded RNA; and
   (b) culturing the cells, and collecting viral particles from the culture supernatant;
(14) a method for producing the vector of (2), wherein the method comprises the steps of:
   (a) introducing a vector expressing the NP protein, a vector expressing the P protein, a vector expressing the L protein, and a vector into which a DNA encoding a *Paramyxoviridae* virus-derived negative single-stranded RNA, or the complementary strand thereof, is inserted, into cells,
      wherein said negative single-stranded RNA has been modified so as not to express multiple proteins selected from the NP, P, and L proteins; and
   (b) culturing the cells, and collecting viral particles from the culture supernatant;
(15) a method for producing the vector of (1), wherein the method comprises the steps of:
   (a) introducing a complex comprising a paramyxovirus-derived negative single-stranded RNA and proteins that bind to said negative single-stranded RNA, into cells expressing the NP, P, L proteins and an envelope protein,
      wherein said negative single-stranded RNA has been modified so as not to express one or more proteins, including at least the L protein selected from the NP, P, and L proteins, which bind to a *Paramyxoviridae* virus negative single-stranded RNA; and
   (b) culturing the cells, and collecting viral particles from the culture supernatant;
(16) a method for producing the vector of (2), wherein the method comprises the steps of:
   (a) introducing a complex comprising a paramyxovirus-derived negative single-stranded RNA and proteins that bind to said negative single-stranded RNA, into cells expressing the NP, P, L proteins and an envelop protein,
      wherein said negative single-stranded RNA has been modified so as not to express multiple proteins selected from the NP, P, and L proteins, which bind to a *Paramyxoviridae* virus negative single-stranded RNA; and
   (b) culturing the cells, and collecting viral particles from the culture supernatant;
(17) a method for producing a vector for expressing a foreign gene in target cells, wherein the method comprises the steps of:
   (a) introducing a DNA encoding a *Paramyxoviridae* virus-derived negative single-stranded RNA, or the complementary strand thereof, into cells capable of expressing the NP, P, and L proteins,
      wherein said negative single-stranded RNA has been modified so as not to express one or more proteins, including at least the L protein selected from the NP, P, and L proteins, and carries a foreign gene; and
   (b) culturing the cells, and collecting viral particles from the culture supernatant;
(18) a method for producing a vector for expressing a foreign gene in target cells, wherein the method comprises the steps of:
   (a) introducing a DNA encoding a *Paramyxoviridae* virus-derived negative single-stranded RNA, or the complementary strand thereof, into cells capable of expressing the NP, P, and L proteins,
      wherein said negative single-stranded RNA has been modified so as not to express multiple proteins selected from the NP, P, and L proteins, and carries a foreign gene; and
   (b) culturing the cells, and collecting viral particles from the culture supernatant;
(19) a method for expressing a foreign gene in target cells, wherein the method comprises the steps of:
   (a) producing the vector of (7); and
   (b) introducing the vector of (7) into target cells.

### Mode for Carrying Out the Invention

The present invention provides novel non-replicating vectors based on *Paramyxoviridae* viruses.

Herein, "paramyxovirus" refers to viruses belonging to *Paramyxoviridae* and derivatives thereof. *Paramyxoviridae* is a group of viruses that have a non-segmented negative-strand RNA as the genome, and includes *Paramyxovirinae* (including the genera *Respirovirus* (also referred to as *Paramyxovirus*), *Rubulavirus*, and *Morbillivirus*) and *Pneumovirinae* (including the genera *pneumovirus* and *metapneumovirus*). Specifically, viruses belonging to the *Paramyxoviridae* family include Sendai virus, Newcastle disease virus, mumps virus, measles virus, respiratory syncytial virus (RS virus), rinderpest virus, distemper virus, simian parainfluenza virus (SV5), and human parainfluenza viruses-1, -2, and -3, etc. More specifically, examples of the *Paramyxoviridae* viruses include: Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV), etc. The viruses of the present invention are preferably viruses belonging to the *Paramyxovirinae* family (including the genera *Respirovirus, Rubulavirus,* and *Morbillivirus*) or derivatives thereof, more preferably viruses belonging to the genus *Respirovirus* (also referred to as the genus *Paramyxovirus*) or derivatives thereof. The derivatives include viruses that are genetically modified or chemically modified in a manner that does not impair their gene-transferring ability. Examples of viruses of the genus *Respirovirus* that can be applied to the present invention include: human parainfluenza virus-1 (HPIV-1), human parainfluenza virus-3 (HPIV-3), bovine parainfluenza virus-3 (BPIV-3), Sendai virus (also referred to as murine parainfluenza virus-1), measles virus, simian parainfluenza virus (SV5), and simian parainfluenza virus-10 (SPIV-10), etc. A more preferred paramyxovirus in the present invention is Sendai virus.

In general, a paramyxovirus contains within its envelope a complex (ribonucleoprotein (RNP)) consisting of RNA and proteins. The RNA comprised in the RNP is the genome of paramyxovirus, which is a minus-strand (negative-strand) single-stranded RNA. This single-stranded RNA binds to the NP, P, and L proteins to form an RNP. The RNA comprised in the RNP serves as the template for transcription and replication of the viral genome (Lamb, R. A., and D. Kolakofsky, 1996, Paramyxoviridae: The viruses and their replication. pp. 1177-1204. In Fields Virology, 3rd edn. Fields, B. N., D. M. Knipe, and P. M. Howley et al. (ed.), Raven Press, New York, N. Y.).

"NP, P, M, F, HN, and L genes" of Paramyxovirus refers to genes encoding the nucleocapsid, phospho, matrix, fusion, hemagglutinin-neuraminidase, and large proteins, respectively. The nucleocapsid (NP) protein binds to the genomic RNA and is essential for the template activity of the genomic RNA. In general, the NP gene is occasionally referred to as the "N gene". The phospho (P) protein is a phosphorylated protein, which is a small subunit of RNA polymerase. The matrix (M) protein functions to support the viral particle structure from the inside. The fusion (F) protein is a membrane fusion protein involved in invasion into host cells. The hemagglutinin-neuraminidase (HN) protein is involved in adhesion to host cells. The large (L) protein is a large subunit of RNA polymerase. Each of the genes described above has an independent transcription regulatory unit. Thus, each gene is transcribed into an individual mRNA, which in turn is translated into a protein. The P gene is translated into the P protein, as well as a non-structural protein (C) by using an ORF other than that of the P protein, and another protein (V) produced via RNA editing of the P protein mRNA during translation. In general, respective genes of viruses belonging to the *Paramyxovirinae* family are shown below (starting from the 3' end).
The genus *Respirovirus*: N P/C/V M F HN - L
The genus *Rubulavirus*: N P/V M F HN (SH) L
The genus *Morbillivirus*: N P/C/V M F H - L

For example, the database accession numbers for the nucleotide sequences of the Sendai virus genes are M29343, M30202, M30203, M30204, M51331, M55565, M69046, and X17218 for the N gene; M30202, M30203, M30204, M55565, M69046, X00583, X17007, and X17008 for the P gene; D11446, K02742, M30202, M30203, M30204, M69046, U31956, X00584, and X53056 for the M gene; D00152, D11446, D17334, D17335, M30202, M30203, M30204, M69046, X00152, and X02131 for the F gene; D26475, M12397, M30202, M30203, M30204, M69046, X00586, X02808, and X56131 for the HN gene; and D00053, M30202, M30203, M30204, M69040, X00587, and X58886 for the L gene.

Examples of viral genes encoded by other viruses are CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for the N gene; CDV, X51869; DMV, Z47758; HPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for the P gene; CDV, AF014953; DMV, Z47758; HPIV-1. M74081; HPIV-3, D00047; MV, AB016162; RPV, X68311; SeV, AB005796; and Tupaia, AF079780 for the C gene; CDV, M12669; DMV Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for the M gene; CDV, M21849; DMV, AJ224704; HPN-1, M22347; HPIV-2, M60182; HPIV-3, X05303, HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for the F gene; CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, X74443; PDV, Z36979; RPV, AF132934; SeV, U06433; and SV-5, S76876 for the HN (H or G) gene; and CDV, AF014953; DMV, AJ608288; HPIV-1, AF117818; HPIV-2, X57559; HPIV-3, AB012132; Mumps, AB040874; MV, K01711; NDV, AY049766; PDPR, AJ849636; PDV, Y09630; RPV, Z30698; and SV-5, D13868 for the L gene.

Multiple strains are known for each virus, and depending on the strain, there are genes comprised of sequences other than the examples listed above.

The non-replicating vectors of the present invention (hereinafter referred to as "the vectors of the present invention") lack self-replicating ability, since the negative single-stranded RNA has been modified so as not to express some or all of the proteins that bind to the negative single-stranded RNA. Herein, "proteins that bind to a negative single-stranded RNA" refers to proteins that form a complex with a negative single-stranded RNA by directly and/or indirectly binding to the negative single-stranded RNA. The complex of the present invention includes a complex comprising a negative single-stranded RNA derived from a paramyxovirus and proteins derived from a paramyxovirus (the NP, P, and L proteins) that bind to the negative single-stranded RNA. Herein, "derived from a paramyxovirus" means that the paramyxovirus components (including the proteins and RNA) are in the non-modified state or a partially modified state. For example, a protein or RNA prepared by modifying a paramyxovirus protein or RNA is a protein or RNA "derived from a paramyxovirus", respectively. There is no limitation on the type of vectors in the present invention, as long as they have the above-described characteristics. For example, the vectors of the present invention may be viral vectors that take a viral particle structure having envelope proteins (the F, HN, and M proteins) and such. Alternatively, the vectors may be RNP vectors, which are RNPs without the viral envelope.

The NP, P, and L proteins of a *Paramyxoviridae* virus bind to a negative single-stranded RNA and play an essential role in genomic RNA replication and protein expression (hereinafter, the NP, P, and L proteins are occasionally referred to as "genomic RNA-binding proteins"). The NP protein binds very tightly to RNA and thereby confers the genomic RNA with template activity. The genomic RNA has template activity for RNA synthesis only when it binds to the NP protein. The RNA has no template activity when it is free from the NP protein. The P and L proteins bind to the genomic RNA as the small and large subunits of RNA polymerase, respectively. Therefore, in *Paramyxoviridae* viruses, genomic RNA replication cannot occur if any one of the NP, P, and L proteins is defective.

In the first embodiment, a vector of the present invention comprises a complex comprising: (a) a *Paramyxoviridae* virus-derived negative single-stranded RNA that has been modified so as not to express one or more proteins that bind to the negative single-stranded RNA, including at least the L protein selected from the NP, P, and L proteins, which bind to the *Paramyxoviridae* virus negative single-stranded RNA; and (b) the NP, P, and L proteins. The vectors of the present invention are viral particles that comprise a complex (RNP) comprising a modified negative single-stranded RNA (genomic RNA) and the NP, P, and L proteins. In the first embodiment, the negative single-stranded RNA comprised in the vector of the present invention has been modified so as not to express at least the L protein. When hosts are infected with a vector of the present invention, proteins are expressed from the genes encoded in the genomic RNA by action of the NP, P, and L proteins comprised in the vector of the present invention. However, genes for the genome-binding proteins including the L protein are not expressed, since they are not encoded in the genomic RNA of the vector of the present invention. Thus, the vector cannot form any self-replicating viral particle, which contains the genomic RNA and NP, P, and L proteins. Specifically, the vectors of the present invention are non-replicating viral vectors. When host cells are infected with a vector of the present invention carrying a foreign gene, the foreign gene is expressed in the host cells; however, no self-replicating viral particle is produced from the vectors of the present invention.

Thus, the vectors of the present invention are highly safe in that they have no self-replicating ability. If the self-replicating ability is to be simply eliminated, the purpose can be achieved by introducing mutations that would impair functions of the NP, P, and L proteins into the NP, P, and L genes in the genomic RNA. However, the significance is that the vectors of the present invention eliminate protein expression, instead of introducing mutations into genes to express proteins that are functionally deficient. That is, the vectors of the present invention are safer than simple non-replicating vectors because of their reduced immunogenicity. In view of the above, the genomic RNA comprised in the vectors of the present invention may lack only the L gene. However, the genomic RNA preferably lacks the NP or P gene in addition to the L gene, and more preferably all of the NP, P, and L genes.

The vectors of the present invention have the advantages described below, because the L gene, of all the RNA-binding protein genes, has been deleted from the genome. Since the length of the L gene is about half of the genomic RNA, the size of the genomic RNA of the vectors of the present invention is significantly reduced due to deletion of the L gene. Thus, the first advantage of the vectors of the present invention is that they can carry longer foreign genes as compared to when other genes are deleted. The second advantage of the vectors of the present invention is that higher transcription efficiency and higher expression levels are expected due to reduction of the genome size. In particular, when a foreign gene is expressed using a non-replicating viral vector, increase in the expression level of the foreign gene via self-replication of the viral vector is not anticipated; thus, higher transcription efficiency is important for securing higher protein expression levels.

In the second embodiment, the vector of the present invention comprises a complex comprising: (a) a *Paramyxoviridae* virus-derived negative single-stranded RNA that has been modified so as not to express multiple proteins selected from proteins that bind to a *Paramyxoviridae* virus negative single-stranded RNA; and (b) the NP, P, and L proteins. In the second embodiment, the genomic RNA of the viral vector includes, for example, without being limited thereto, a genomic RNA that encodes the L gene but has been modified so as not to encode the two genes, NP and P. The genomic RNA also includes all of the genomic RNAs that have been modified so as not to express two or three of the genomic RNA-binding proteins. By deleting multiple genes, the amount of virus-derived proteins is reduced as compared to when either one of the NP and P genes is deleted. In addition, since the length of the genomic RNA is reduced, the vector can carry larger foreign genes.

As described above, the genomic RNA of the vectors of the present invention lacks some or all of the genes for the genomic RNA-binding proteins. In the first embodiment, the genomic RNA lacks at least the L gene, preferably the L and NP genes, or the L and P genes, and more preferably all of the NP, P, and L genes. In the second embodiment, the genomic RNA lacks multiple genes for the genomic RNA-binding proteins. Meanwhile, the sequences of the genes encoded by the genomic RNA may be the original gene sequences derived from a virus or may have some mutations introduced. For example, those skilled in the art can introduce into genes in the genomic RNA, mild mutations that do not cause any functional impairment on proteins, by known methods. For example, site-specific mutations can be introduced by the PCR method, cassette mutagenesis method, or such. Alternatively, random mutations can be introduced by using chemical reagents, random nucleotides, or such. The stringent hybridization conditions described above can be appropriately selected by those skilled in the art. Such conditions include, for example, overnight prehybridization at 42°C followed by overnight hybridization at 42°C, using a hybridization solution containing 25% formamide, 4x SSC, 50 mM Hepes (pH 7.0), 10x Denhardt's solution, and 20 µg/ml denatured salmon sperm DNA, or using a hybridization solution containing 50% formamide, 4x SSC, 50 mM Hepes (pH 7.0), 10x Denhardt's solution, and 20 µg/ml denatured salmon sperm DNA for more stringent conditions. Post-hybridization wash may be carried out using the washing solution and temperature conditions of "1x SSC, 0.1% SDS, 37°C" or such, "0.5x SSC, 0.1% SDS, 42°C" or such for more stringent conditions, or "0.2x SSC, 0.1% SDS, 65°C" or such for even more stringent conditions. Polypeptides encoded by polynucleotides isolated using such hybridization techniques will usually share a high amino acid sequence identity with polypeptides encoded by the above-described negative-strand RNA. "High identity" refers to a sequence identity of at least 40% or more, preferably 60% or more, more preferably 80% or more, even more preferably 90% or more, still more preferably at least 95% or more, and yet more preferably at least 97% or more (for example, 98% to 99%). Amino acid sequence identity can be determined, for example, using the BLAST algorithm by Karlin and Altschul (Proc. Natl. Acad. Sci. USA 87:2264-2268, 1990; Proc. Natl. Acad. Sci. USA 90:5873-5877, 1993). When amino acid sequences are analyzed using BLASTX developed based on this algorithm (Altschul et al. J. Mol. Biol. 215:403-410, 1990), the parameters are set, for example, as follows: score = 50 and wordlength = 3. When the BLAST and Gapped BLAST programs are used, the default parameters of each program may be used. Specific procedures of these analytical methods are known (http://www.ncbi.nlm.nih.gov.).

Furthermore, it is known that the cytotoxicity of a paramyxovirus vector can be reduced by introducing mutations into the P gene. When the P gene is encoded by genomic RNA of the vector of the present invention, such mutations may be introduced into the RNA gene of the genome. Specifically, the mutations include, for example, substitution of a different amino acid for Leu at position 511 (L511) of the SeV P protein, or substitution at the homologous position of the P protein in other negative-strand RNA viruses. The amino acid mutations may be desirable substitutions by a different amino acid, and preferably by amino acids whose side chains have different chemical properties. Amino acids can be categorized, for example, into groups such as basic amino acids (for example, lysine, arginine, and histidine), acidic amino acids (for example, aspartic acid and glutamic acid), non-charged polar amino acids (for example, glycine, asparagine, glutamine, serine, threonine, tyrosine, and cysteine), non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). The amino acid substitutions include substitution of an amino acid by another amino acid different those in the group to which the original amino acid belongs. Specifically, the substitutions include substitution of a basic amino acid by an acidic or neutral amino acid; substitution of a polar amino acid by an non-polar amino acid; substitution of an amino acid whose molecular weight is higher than the average molecular weight of the 20 types of natural amino acids by an amino acid whose molecular weight is lower than the average molecular weight; and substitution of an amino acid whose molecular weight is lower than the average molecular weight by an amino acid whose molecular weight is higher than the average molecular weight; however, the substitutions are not limited to those described above. Specifically, the substitutions include, for example, the substitution of L511 by Phe (L511F).

The genomic RNA comprised in the vectors of the present invention may encode all of the envelope protein genes or may fail to encode some or all of the envelope protein genes. It may be possible to further reduce the immunogenicity of the vectors upon *in vivo* administration by modifying their genomic RNA so as not to express the envelope protein genes in addition to the genomic RNA-binding protein genes. The envelope protein genes (the M, F, and HN genes) encoded by the genomic RNA may be wild type or may have temperature-sensitive mutations introduced. Temperature-sensitive mutations of the envelope proteins are described in detail in WO 2003/025570.

The genomic RNA comprised in the vectors of the present invention lacks at least the L gene, or multiple genes for the genomic RNA-binding proteins. The RNP of the present invention can be produced by transcribing the genomic RNA (positive or negative strand) in the presence of the NP, P, and L proteins. RNP formation can be achieved, for example, using BHK-21 cells, LLC-MK2 cells, or such. The NP, P, and L proteins can be supplied by various methods, as long as the proteins are not supplied by a viral vector. For example, the proteins can be supplied by introducing expression vectors encoding the genes into cells (see the Examples). Alternatively, each gene may be integrated into the host cell chromosome. The NP, P, and L genes expressed to form RNPs are not necessarily completely the same as those encoded by the vector genome. Specifically, amino acid sequences of the proteins encoded by these genes are not necessarily identical to those of the proteins encoded by the RNP genome. Mutations may be introduced into the proteins, or homologous genes of other viruses may be used as substitutes, as long as the proteins bind to the genomic RNA and have the activity of transcribing and replicating the genome in cells.

When the NP, P, and L proteins are expressed in cells at the time of intracellular vector reconstitution, the proteins are incorporated into viral vectors. Thus, viral vectors with infectivity can be produced. Once cells are infected with such vectors, proteins can be expressed from the genomic RNA by intracellular RNPs. However, since the vectors themselves do not have the L gene or such, they cannot reproduce viruses that have the same replication ability as the original virus. Such vectors are very useful for gene therapy and the like, particularly for target diseases and fields of application that require the effects of the vectors to be minimized.

In general, the reconstituted viral vectors of the present invention can be prepared by: (a) introducing a vector DNA that encodes a paramyxovirus-derived negative single-stranded RNA, or the complementary strand thereof, into cells (helper cells) expressing paramyxovirus-derived proteins that bind to the negative single-stranded RNA, wherein the negative single-stranded RNA has been modified so as not to express one or more proteins including the L protein from the paramyxovirus NP, P, and L proteins, and expressing the vector DNA; and (b) culturing the cells, and collecting viral particles from the culture supernatant. Alternatively, it is also possible to use "a DNA that encodes a *Paramyxoviridae* virus-derived negative single-stranded RNA derived or the complementary strand thereof, wherein the negative single-stranded RNA has been modified so as not to express multiple proteins selected from the NP, P, and L proteins" in step (a) above. The virus of the present invention is constituted by co-expressing the NP, L, and P proteins together with the vector DNA, thereby forming an RNP.

The vector cDNA expressed in the helper cells encodes a negative single-stranded RNA (negative strand) comprised in the vector of the present invention, or the complementary strand thereof (positive strand). For example, a DNA encoding a negative single-stranded RNA or the complementary strand thereof is linked downstream of the T7 promoter, to transcribe the DNA into RNA using the T7 RNA polymerase. The vector cDNA may be cloned into a plasmid so that it can be amplified in *E. coli*. The strand that is transcribed in cells may be the positive strand or negative strand of a virus. However, it is preferable to have transcription of the positive strand to increase the reconstitution efficiency. In the Examples described herein, the vector cDNA (SEQ ID NO: 3) is, for example, designed in a way that the positive strand is transcribed.

Cells expressing the NP, L, and P proteins are used as the helper cells. The helper cells are not limited to those expressing the proteins (NP, L, and P) deleted from a viral vector. The helper cells may be those that express different genomic RNA-binding proteins from those encoded by the genes deleted from the viral vector. For example, genomic RNA-binding proteins from a different paramyxovirus can be used as long as they allow replication and transcription of the genomic RNA.

These genes can be expressed in the helper cells by various methods, as long as they do not use viral vectors. The viral vector can be reconstituted, for example, by co-transfecting host cells with a plasmid expressing a recombinant Sendai virus vector genome that lacks the L gene, or multiple genes including the L gene, together with expression vectors for the NP, P/C, and L proteins. Alternatively, the viral vector can be produced, for example, by using host cells in which the L gene is integrated into the chromosome. The proteins supplied from a source other than the viral genome do not necessarily have identical amino acid sequences as those derived from the original virus. Mutations may be introduced into the proteins, or homologous genes of other viruses may be used as substitutes, as long as the activity that results from introduction of the nucleic acids is equivalent to or higher than the activity of the natural proteins.

The helper cells may be cells that further express proteins having the function to promote formation and release of viral particles, for example, the C protein. The C protein is preferably expressed in the helper cells to achieve high productivity of the viral vectors of the present invention. The Sendai virus C protein includes four types of proteins, C', C, Y1, and Y2, which are translated using different initiation codons in the same reading frame. Any one or two or more of the above-mentioned four types of C protein may be expressed in the helper cells at the time of reconstituting the viral vectors of the present invention. Helper cells expressing all four types, C', C, Y1, and Y2, are preferably used for reconstituting the viral vectors of the present invention. Deletion of the P gene from the genomic RNA of the viral vector of the present invention results in deletion of the C gene which is in a different reading frame from that of the P gene. When the genomic RNA lacks the C gene, the C gene can be expressed in helper cells by introducing an expression vector encoding the C gene into the helper cells. However, it can be difficult to supply the C protein by constitutive expression, since replication of the vector genome is known to be suppressed in cell lines constitutively expressing the C protein (Kato A, et al., J Virol. 78, 7443-54 (2004)). For an example of methods for expressing the C protein in helper cells, one can refer to the Examples described below.

Once RNPs or viruses are formed, the vectors of the present invention can be amplified by introducing the RNPs or viruses into the above-described helper cells and culturing them. This process comprises the steps of:
(a) introducing a complex comprising a paramyxovirus-derived negative single-stranded RNA, and proteins that bind to the negative single-stranded RNA, into cells expressing an envelope protein, wherein the negative single-stranded RNA has been modified so as not to express one or more proteins, including at least the L protein selected from the NP, P, and L proteins, which bind to a *Paramyxoviridae* virus negative single-stranded RNA; and
(b) culturing the cells, and collecting viral particles from the culture supernatant.

Alternatively, the process comprises the steps of:
(a) introducing a complex comprising a paramyxovirus-derived negative single-stranded RNA, and proteins that bind to the negative single-stranded RNA, into cells expressing the NP, P, and L proteins and an envelope protein, wherein the negative single-stranded RNA has been modified so as not to express multiple proteins selected from the NP, P, and L proteins, which bind to a *Paramyxoviridae* virus negative single-stranded RNA; and
(b) culturing the cells, and collecting viral particles from the culture supernatant.

RNP may be introduced to cells as a complex formed together with, for example, lipofectamine and a polycationic liposome. Specifically, a variety of transfection reagents can be utilized. Examples thereof are DOTMA (Boehringer), Superfect (QIAGEN #301305), DOTAP, DOPE, DOSPER (Boehringer #1811169), Lipofectamine 2000 (Invitrogen), etc. Chloroquine may be added to prevent RNP from decomposition in endosomes (Calos, M. P., 1983, Proc. Natl. Acad. Sci. USA 80: 3015).

esides a viral envelope protein, for example, a chimeric protein comprising, in its extracellular region, a polypeptide derived from an adhesion molecule, ligand, receptor protein, and such that can adhere to specific cells, and in its intracellular region, polypeptides derived from virus envelope can be used as an envelope protein. Hereby, vectors targeted to specific tissues can be produced. The viral vectors of the present invention, for example, may comprise a viral gene contained in a vector that has been modified to reduce the antigenicity or enhance the RNA transcription and replication efficiencies.

The vectors of the present invention may include RNA encoding a foreign gene in their negative single-stranded RNA. Any desirable gene to be expressed in target cells may be used as the foreign gene. For example, when gene therapy is intended, a gene for treating a disease of interest is inserted into the viral vector gene. In the case where a foreign gene is inserted into the viral vector gene, for example, the Sendai viral vector gene, it is necessary to consider the total number of nucleotides in the genome to be a multiple of six, and insert a sequence between the transcription initiation sequence (S) and transcription termination sequence (E), or such. (Calain, P. and Roux, L., Journal of Virology, Vol. 67, No. 8, 1993, p.4822-4830). The foreign gene may be inserted before or after each of the virus genes (see the Examples). An E-I-S sequence (transcription termination sequence-intervening sequence-transcription initiation sequence) or portion thereof is appropriately inserted before or after a foreign gene so as not to interfere with expression of the genes before or after the foreign gene. Expression level of the inserted foreign gene can be regulated by the type of transcription initiation sequence added upstream of the foreign gene, as well as the site of gene insertion and nucleotide sequences before and after the gene. For example, in Sendai virus, the closer the insertion site is to the 3'-end of negative-strand RNA (closer to NP gene in the gene arrangement on the wild-type viral genome), the higher the expression level of the inserted gene is. To secure a high expression level of a foreign gene, it is preferable to insert the foreign gene into an upstream region in the negative-strand genome (the 3'-side in the negative-strand). Conversely, the closer the insertion position is to the 5'-end of negative-strand RNA (closer to the L gene in the gene arrangement on the wild-type viral genome), the lower the expression level of the inserted gene is. To suppress the expression of a foreign gene to a low level, the foreign gene is inserted, for example, to the far most 5'-side of the negative-strand, that is, downstream of the L gene in the wild-type viral genome (the 5'-side adjacent to the L gene in the negative-strand) or upstream of the L gene (the 3'-side adjacent to the L gene in the negative-strand).

One or more genomic promoters may be inserted together with a foreign gene. In the present invention, the genomic promoters include, for example, the RNA polymerase I promoter, without being particularly limited thereto. When cells expressing RNA polymerase I are used as production cells, it is unnecessary to supply RNA polymerase I in a foreign vector, and thus, the supply cost can be reduced. In addition, as with the T7 system, high-efficiency gene expression can be achieved.

To facilitate the insertion of a foreign gene, a cloning site may be designed at the position of insertion. The cloning site can be made into, for example, a recognition sequence for restriction enzyme. Foreign gene fragments can be inserted into the restriction enzyme site in the vector DNA encoding the genome. Cloning site may be arranged to be a so-called multi-cloning site comprising a plurality of restriction enzyme recognition sequences. Vectors of the present invention may thus harbor additional foreign genes.

Recombinant Sendai virus vectors comprising a foreign gene can be constructed as follows, according to, for example, the description in Kato, A. et al., 1997, EMBO J. 16: 578-587, and Yu, D. et al., 1997, Genes Cells 2: 457-466.

First, a DNA sample comprising the cDNA nucleotide sequence of a desired foreign gene is prepared. It is preferable that the DNA sample can be electrophoretically identified as a single plasmid at a concentration of 25 ng/µl or more. Hereinbelow, a case where a foreign gene is inserted to a DNA encoding a viral genome utilizing the *Not*I site will be described as an example. When the *Not*I recognition site is included in the cDNA nucleotide sequence of interest, it is preferable to delete the *Not*I site beforehand by modifying the nucleotide sequence using site-specific mutagenesis and such so as not to alter the amino acid sequence encoded by the cDNA. From this DNA sample, the desired gene fragment is amplified and recovered by PCR. To have *Not*I sites on both ends of the amplified DNA fragment, and further add a copy of the transcription termination sequence (E), intervening sequence (I) and transcription initiation sequence (S) (EIS sequence) of Sendai virus to one end, a forward synthetic DNA sequence (sense strand) and reverse synthetic DNA sequence (antisense strand) are prepared as a pair of primers containing the *Not*I restriction enzyme cleavage site sequence, the transcription termination sequence (E), intervening sequence (I), and transcription initiation sequence (S), and a partial sequence of the gene of interest.

For example, to secure cleavage by *Not*I, the forward synthetic DNA sequence is arranged in a form, in which any two or more nucleotides (preferably four nucleotides excluding GCG and GCC, which are sequences originating in the *Not*I recognition site, more preferably ACTT) are selected on the 5'-side of the synthetic DNA, the *Not*I recognition site "gcggccgc" is added to its 3'-side, and to the 3'-side thereof, any nine nucleotides or nucleotides of nine plus a multiple of six nucleotides are added as the spacer sequence, and to the 3'-side thereof, a sequence of about 25 nucleotides from the ORF including the initiation codon ATG of the desired cDNA is added. It is preferable to select about 25 nucleotides from the desired cDNA as the forward side synthetic DNA sequence so that it has G or C as the final nucleotide on its 3'-end.

In the reverse synthetic DNA sequence, any two or more nucleotides (preferably four nucleotides excluding GCG and GCC, which are sequences originating in the *Not*I recognition site, more preferably ACTT) are selected from the 5'-side of the synthetic DNA, and the *Not*I recognition site "gcggccgc" is added to its 3'-side. Further to its 3'-side, an oligo DNA is added as an insertion fragment to adjust the length. The number of nucleotides in the oligo DNA is determined so that the total nucleotide number, of the Sendai virus genome becomes a multiple of six (so-called "rule of six"; Kolakofski, D. et al., J. Virol. 72: 891-899, 1998). Further to the 3'-side of inserted fragment, sequences complementary to the S sequence of Sendai virus, preferably 5'-CTTTCACCCT-3' (SEQ ID NO: 8), the I sequence, preferably 5'-AAG-3', and the E sequence, preferably 5'-TTTTTCTTACTACGG-3' (SEQ ID NO: 9), are added. Further to the 3'-side, a complementary sequence of about 25 nucleotides counted in the reverse direction from the termination codon of the desired cDNA sequenceis selected and added as the 3'-end of the reverse synthetic DNA, wherein the length of the complementary sequence is adjusted so that the final nucleotide is G or C.

PCR can be performed according to conventional methods with, for example, ExTaq polymerase (Takara Shuzo). Preferably, PCR is performed using Vent polymerase (NEB), and the fragments of interest thus amplified are digested with *Not*I, then inserted to the *Not*I site of the plasmid vector pBluescript. Nucleotide sequences of the PCR products thus obtained are confirmed with a sequencer to select a plasmid having the correct sequence. The inserted fragment is excised from the plasmid using *Not*I, and cloned to the *Not*I site of a plasmid carrying the genomic cDNA deficient in envelope genes. Alternatively, it is also possible to obtain a recombinant Sendai virus cDNA by directly inserting the fragment to the *Not*I site without mediation of the plasmid vector pBluescript.

The viral vector cDNA of the present invention is transcribed *in vitro* or in cells, and is allowed to bind to separately expressed L, P, and NP proteins to reconstitute an RNP. Thus, viral vectors comprising the RNP can be produced. According to known methods (WO 97/16539; WO 97/16538; Durbin, A.P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M.D. and Barrett, T., 1997, J. Virol. 71: 1265-1271; Bridgen, A. and Elliott, R.M., 1996, Proc. Natl. Acad. Sci. USA 93: 15400-15404), viral particles can be reconstituted from a viral vector cDNA encoding a negative single-stranded RNA, which has been modified so as not to express some of the virus-derived proteins, or the complementary strand thereof. In particular, improved methods are effective (WO 2005/071092). Genes that have been deleted from the viral vector cDNA of the present invention can be expressed by introducing them into host cells separately. When viral particles are reconstituted by forming RNPs and expressing envelope proteins using genes encoded by the viral vector cDNA of the present invention or separate expression vectors, the viral particles can be infectious.

Alternatively, an RNP complex itself can be introduced into cells as an RNA vector, by forming a complex with Lipofectamine, polycationic liposomes, or such, as described above.

Methods for introducing viral vector cDNAs into cells include: (i) methods of preparing DNA precipitates that can be internalized into the cells of interest; (ii) methods of preparing positively-charged complexes comprising DNAs that are suitable for internalization into the cells of interest, and have low cytotoxicity; and (iii) methods of instantaneously creating holes with a size sufficient for DNA molecules to pass through on the membrane of cells of interest using electric pulses.

Various transfection reagents can be used in the methods of (ii). Such reagents include, for example, DOTMA (Boehringer), Superfect (QIAGEN #301305), DOTAP, DOPE, DOSPER (Boehringer #1811169), and Lipofectamine 2000 (Invitrogen). The methods of (i) include, for example, transfection methods using calcium phosphate. It is known that while DNA incorporated into cells by these methods is internalized into phagosomes, a sufficient amount of the DNA also enter the nucleus (Graham, F. L. and Van Der Eb, J., 1973, Virology 52: 456; Wigler, M. and Silverstein, S., 1977, Cell 11: 223). Chen and Okayama studied the optimization of transfer techniques, and reported that (1) incubation conditions for cells and co-precipitates are: 2 to 4% CO₂ at 35°C for 15 to 24 hours; (2) the activity of circular DNAs is higher than that of linear DNAs; and (3) optimal precipitate can be obtained when the DNA concentration of precipitation mixture is 20 to 30 µg/ml (Chen, C. and Okayama, H., 1987, Mol. Cell. Biol. 7: 2745). The methods of (ii) are suitable for transient transfection. Methods for performing transfection by preparing a DEAE-dextran (Sigma #D-9885 M.W. 5 x 10⁵) mixture with a desired DNA concentration ratio have been known for a long time. Since most complexes are degraded in endosomes, chloroquine may be added to enhance the effect (Calos, M.P., 1983, Proc. Natl. Acad. Sci. USA 80: 3015). The methods of (iii) are referred to as electroporation methods, which are not cell-selective, and thus have high versatility compared to the methods of (i) and (ii). The efficiency of these methods is assumed to be high under optimal conditions for the duration of pulse electric current, shape of the pulse, potency of electric field (gap between electrodes, voltage), buffer conductivity, DNA concentration, and cell density.

Of the above three categories, the methods of (ii) are simpler to perform and can be used to examine many samples with a large number of cells. Transfection reagents that can be suitably used in the present invention include Lipofectamine 2000 (Invitrogen), Superfect Transfection Reagent (QIAGEN, Cat No. 301305), and DOSPER Liposomal Transfection Reagent (Boehringer Mannheim, Cat No. 1811169).

Titers of collected viruses can be determined, for example, by measuring CIU (Cell Infectious Unit) or Hemagglutination Activity (HA) (WO 00/70070; Kato, A. et al., 1996, Genes Cells 1: 569-579; Yonemitsu, Y. & Kaneda, Y., Hemagglutinating virus of Japan-liposome-mediated gene delivery to vascular cells. Ed. by Baker AH. Molecular Biology of Vascular Diseases. Method in Molecular Medicine: Humana Press: pp. 295-306, 1999). Alternatively, titers of vectors carrying marker genes such as GFP (green fluorescent protein) can be quantified by directly counting infected cells, using the marker as an index (for example, as GFP-CIU). Titers thus determined can be treated in the same way as CIU (WO 00/70070).

The collected paramyxovirus vectors can be purified to be substantially pure. Purification can be achieved using known purification/separation methods including filtration, centrifugation, column purification, and such, or any combination thereof. "Substantially pure" means that the viral vector is a component that accounts for the major proportion of a sample containing the vector. Typically, a viral vector can be confirmed to be substantially pure when the percentage of the viral vector-derived proteins relative to the total proteins (excluding proteins added as carriers and stabilizers) in a sample is 10% or more, preferably 20% or more, more preferably 50% or more, preferably 70% or more, more preferably 80% or more, and even more preferably 90% or more. Specific methods for purifying paramyxoviruses include, for example, methods using cellulose sulfate esters or cross-linked polysaccharide sulfate esters (Japanese Patent Application Kokoku Publication No. (JP-B) S62-30752 (examined, approved Japanese patent application published for opposition), JP-B S62-33879, and JP-B S62-30753), and methods of adsorbing viruses to fucose sulfate-containing polysaccharides and/or degradation products thereof (WO97/32010).

The vectors of the present invention are non-replicating. In addition, since the vectors do not express the L protein or such, the total expression of virus-derived proteins is significantly reduced. Thus, the vectors of the present invention are highly safe. In particular, when used for gene therapy, vectors cannot be repeatedly administered if their immunogenicity is high. In contrast, the vectors of the present invention have low immunogenicity, and are thus expected to widen the applicability of viral vectors in gene therapy.

The vectors of the present invention can be prepared as compositions according to the purpose. When producing compositions comprising the vectors, the vectors may be combined with desired pharmaceutically acceptable carriers or media as necessary. "Pharmaceutically acceptable carriers or media" refers to materials that can be administered together with the vectors and which do not significantly inhibit the vector-mediated gene transfer. For example, such compositions can be prepared by adequately diluting the vectors with physiological saline, phosphate-buffered saline (PBS), or the like. Furthermore, compositions comprising the vectors may also contain carriers or media such as deionized water or aqueous solution of 5% dextrose. The compositions may further contain vegetable oils, suspending agents, surfactants, stabilizers, biocidal agents, and the like. Furthermore, preservatives and other additives may also be added. Compositions comprising the vectors of the present invention are useful as reagents or pharmaceutical agents.

When the vectors are prepared using a gene for treating diseases as the foreign gene, gene therapy can be performed by administering the vectors. When applying the viral vectors of the present invention in gene therapy, foreign genes that are expected to produce therapeutic effects, or endogenous genes that are insufficiently supplied in the patient's body, or the like, can be expressed by gene expression methods, either via direct administration or indirect (*ex vivo*) administration. There is no particular limitation on the foreign gene. In addition to protein-coding nucleic acids, the foreign gene may be, for example, a non-protein-coding nucleic acid such as antisense nucleic acids and ribozymes.

When genes encoding bacterial or viral antigens involved in infection are used as the foreign gene, immunity can be induced in animals by administering the genes into the animals. That is, the genes can be used as vaccines.

When used as vaccines, the vectors of the present invention can be applied to, for example, tumors, infections, and other general diseases. As anti-tumor therapy, for example, genes that have therapeutic effects can be expressed in tumor cells or antigen-presenting cells (APC) such as DC cells, using the vectors of the present invention. Such genes include the cancer antigen Muc-1, the Muc-1-like mucin tandem repeat peptide (U.S. Patent No. 5,744,144), and the melanoma antigen gp100. Treatment using such genes has been shown to be widely applicable to breast cancer, colon cancer, pancreatic cancer, prostate cancer, lung cancer, and such. It is effective to combine such genes with cytokines that enhance the adjuvant effects. Such genes include, for example, (i) the combination of IL-2 and single-chain IL-12 (Proc. Natl. Acad. Sci. USA 96 (15): 8591-8596, 1999); (ii) IL-2 and interferon-γ (U.S. Patent No. 5,798,100); (iii) granulocyte colony stimulating factor (GM-CSF) used alone; and (iv) the combination of GM-CSF and IL-4 for treating brain tumor (J. Neurosurgery 90 (6), 1115-1124 (1999)).

Targets of the treatment of infection include, for example, the envelope of the highly-virulent H5N1 strain for influenza; an envelope chimera for Japanese encephalitis (Vaccine, vol. 17, No. 15-16, 1869-1882 (1999)); the HIV gag and SIV gag proteins (J. Immunology (2000) vol. 164, 4968-4978), as well as the HIV envelope proteins, which are orally administered in vaccination treatment, or encapsulated in polylactate-glycol copolymers for administration (Kaneko, H. et al., Virology 267: 8-16 (2000)) for AIDS; the cholera toxin B subunit (CTB) (Arakawa T, et al., Nature Biotechnology (1998) 16(10): 934-8; Arakawa T, et al., Nature Biotechnology (1998) 16(3): 292-7) for cholera; the rabies virus glycoprotein (Lodmell DL et al., 1998, Nature Medicine 4(8):949-52) for rabies; and the capsid protein L1 of human papilloma virus type 6 (J. Med. Virol, 60, 200-204 (2000)) for cervical carcinoma.

The dosage of the vectors can be appropriately determined by those skilled in the art, although it varies depending on the disease, patient's weight, age, sex, symptom, objective of administration, form of composition administered, administration method, gene to be introduced, and such. The route of administration can be appropriately selected, and includes, for example, percutaneous, intranasal, transbronchial, intramuscular, intraperitoneal, intravenous, intraarticular, intraspinal, and subcutaneous administrations, but is not limited thereto. Intranasal administration is a preferred administration route. The administration may be local or systemic. It is preferred to administer the vectors at a dosage within the range of preferably about 10⁵ CIU/ml to about 10¹¹ CIU/ml, more preferably about 10⁷ CIU/ml to about 10⁹ CIU/ml, and still more preferably about 1 x 10⁸ CIU/ml to about 5 x 10⁸ CIU/ml, together with a pharmaceutically acceptable carrier. A single dose for human is preferably 2 x 10⁵ CIU to 2 x 10¹⁰ CIU. The frequency of administration can be once or more, and within the range of clinically acceptable side effects. The same applies to the daily administration frequency. For protein preparations produced using vectors of the present invention, the protein dosage may be, for example, within the range of 10 ng/kg to 100 µg/kg, preferably 100 ng/kg to 50 µg/kg, and more preferably 1 µg/kg to 5 µg/kg. For non-human animals, for example, the dosage to be administered can be converted from the above-described dosage based on the body weight ratio or volume ratio (*e*.*g*., average value) of the target site for administration between the animal of interest and human. The subject to which a composition comprising a vector of the present invention is administered include all mammals such as human, monkey, mouse, rat, rabbit, sheep, cattle, dog, etc.

All the prior-art documents cited in the present specification are incorporated herein by reference.

### Brief Description of the Drawings

Fig. 1 depicts the process for constructing pMiniSeV/M/F/HN and pMiniSeV/GFP/M/F/HN.

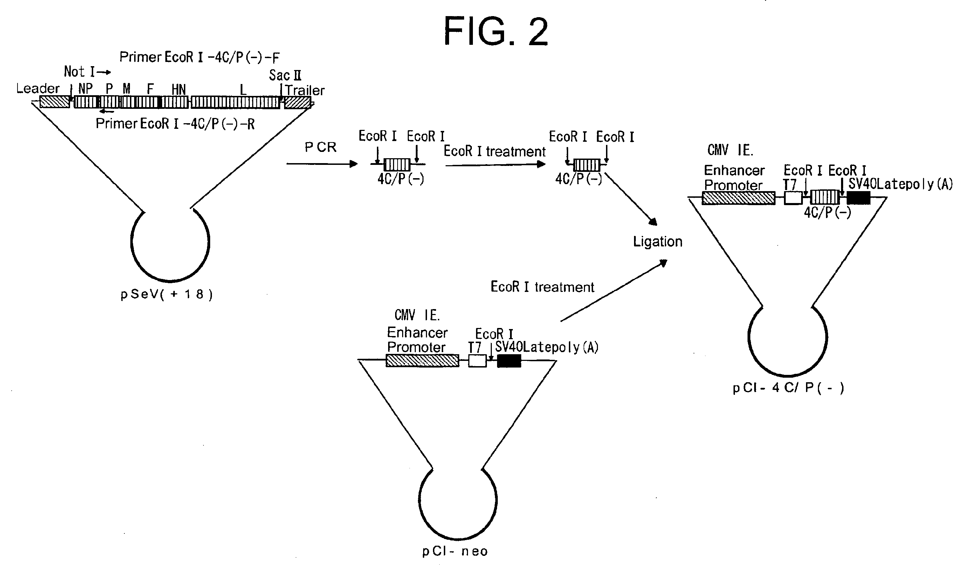

Fig. 2 depicts the process for constructing pCI-4C/P(-).
Fig. 3 presents photographs showing the effect of the SeV C protein on the productivity of MiniSeV/GFP/M/F/HN. (A) Photographs showing GFP fluorescence in production cells four days after infection. BHK-21 cells, into which pCAGGS-NP (Z), pCAGGS-P (Z)/4C(-), pCAGGS-L (TDK), and either pCI control or pCI-4C/P(-) were introduced, were infected with MiniSeV/GFP/M/F/HN. (B) Photographs showing GFP fluorescence in LLC-MK2 cells three days after infection. The cells were infected with the day-four culture supernatant of (A).
Fig. 4 (A) Photographs of GFP imaging showing the productivity of MiniSeV/GFP/M/F/HN one to seven days after infection. (B) A graph showing the titers of MiniSeV/GFP/M/F/HN in the culture supernatants collected from day one to day seven.
Fig. 5 presents fluorescence photographs of *in vitro* GFP expression using MiniSeV/GFP/M/F/HN.
Fig. 6 depicts the process for constructing pMiniSeV_{SP}/GFP and pMiniSeV_{SP}/luciferase.
Fig. 7 depicts the process for constructing pMiniSeV_{DP}/GFP and pMiniSeV_{DP}/luciferase.
Fig. 8 depicts the effect of the genomic promoters on the expression level of the MiniSeV vectors.
Fig. 9 presents fluorescence photographs of GFP expression by MiniSeV_{SP}/GFP.

### Examples

Hereinbelow, the present invention is specifically described with reference to the Examples; however, it should not be construed as being limited thereto.

### [Materials and methods]

### 1. Plasmid construction

### 1.1 pMiniSeV/M/F/HN (also referred to as pSeV/ΔNP/ΔP/ΔL)

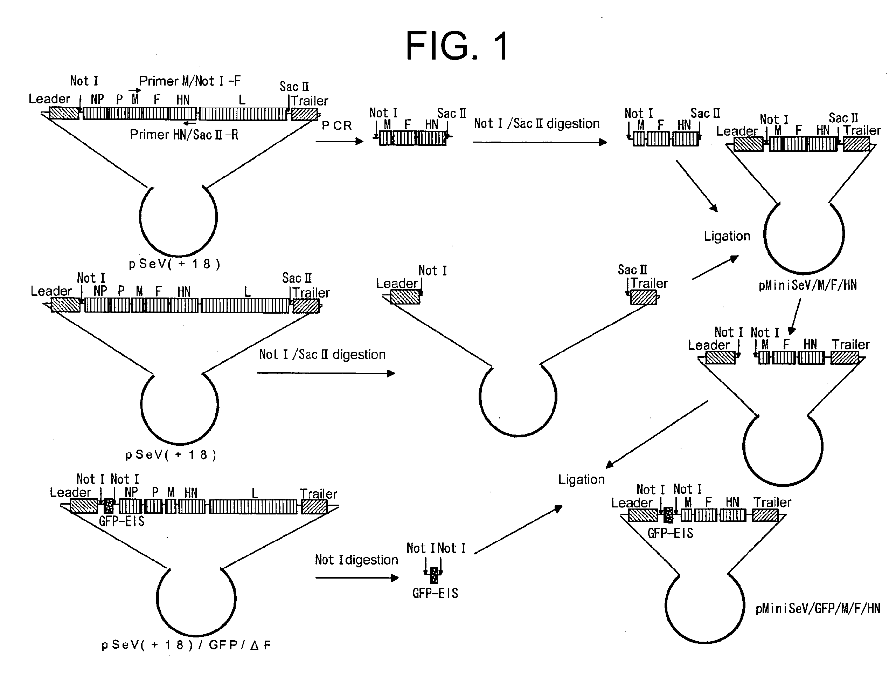

First, a NotI-M/F/HN-SacII fragment was obtained by PCR using pSeV(+18) as a template and the primers M/NotI-F (5'- aagtggcggccgcacggcgcaatggcagatatc, 33mer (SEQ ID NO: 1)) and HN/SacII-R (5'-ggtaccgcggagcttcgatcgttctgcacgatagggactaattattaagactcggccttgcataatttag, 70mer (SEQ ID NO: 2)) (Fig. 1). Next, the fragment was treated with. *Not*I and *Sac*II. After purification, the fragment was inserted into the pSeV (+18) vector, which had been similarly treated with *Not*I and *Sac*II to remove a fragment containing the SeV NP/P/M/F/HN/L sequence. Thus, pMiniSeV/M/F/HN (SEQ ID NO: 3) was obtained.

### 1.2 pMiniSeV/GFP/M/F/HN (also referred to as pSeV/GFP/ΔNP/ΔP/ΔL)

pSeV/GFP/ΔF was treated with *Not*I, and the GFP-EIS/NotI fragment (SEQ ID NO: 4) was separated and purified. Then, the fragment was inserted into the pMiniSeV/M/F/HN vector, which had been similarly treated with *Not*I. Thus, pMiniSeV/GFP/M/F/HN was obtained (Fig. 1). Other genes of interest (GOI) were inserted by the same procedure as that used for the GFP-EIS/NotI fragment sequence. A GOI-EIS/NotI fragment was amplified by PCR, and then treated with *Not*I. After purification, the fragment was inserted into the pMiniSeV/M/F/HN vector, which had been similarly treated with *Not*I. Thus, pMiniSeV/GOI/M/F/HN was obtained.

### 1.3 pCI-4C/P(-)

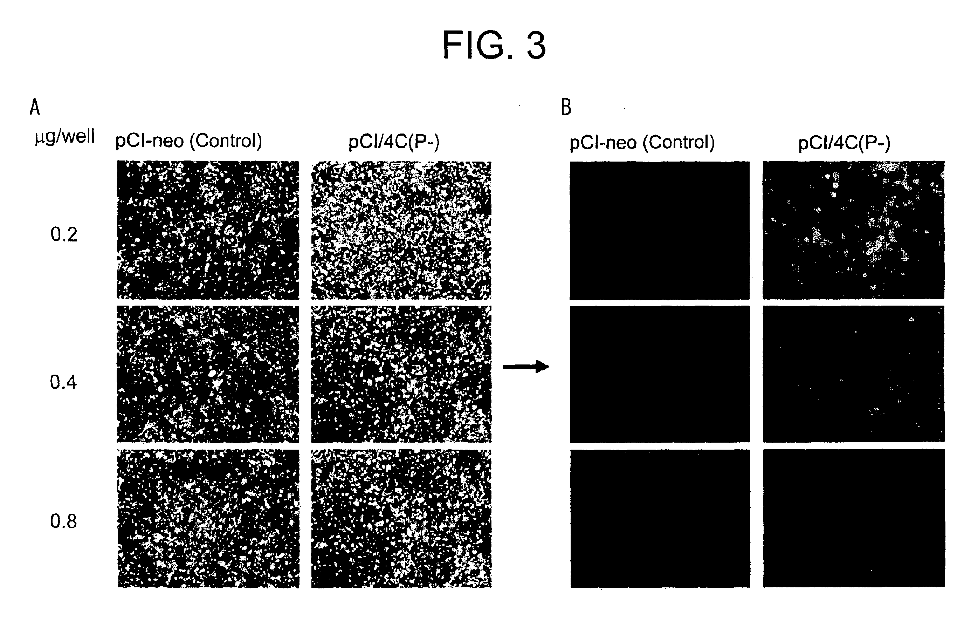

First, an EcoRI-4C/P(-)-EcoRI fragment (SEQ ID NO: 7) was obtained by PCR using pSeV(+18) as a template and the primers EcoRI-4C/P(-)-F (5'-acttgaattcacggcttcggctacacttaccgcctggatcaagatgccttcattc, 55mer (SEQ ID NO: 5)) and EcoRI-4C/P(-)-R (5'- cgaggcggccgcttactcttgcactatgtg, 30mer (SEQ ID NO: 6)) (Fig. 3). Then, the fragment was treated with EcoRI. After purification, the fragment was inserted into the pCI-neo vector, which had been similarly treated with EcoRI and purified. Thus, pCI-4C/P(-) was obtained.

### 2. Reconstitution of MiniSeV/GFP/M/F/HN (also referred to as SeV/GFP/ΔNP/ΔP/ΔL)

2.1 Preparation
(1) Cells: the day before, about 8e5 BHK-21 cells/well (6-well plate)
(2) Opti-MEM [Cat No. 31985-070, Lot No. 1183337, Invitrogen] containing 20% FBS (without P&S)
(3) Opti-MEM (stock solution)

2.2 Preparation of transfection solution
(1) DNA solution (per well)

| | |
|---|---|
| Opti-MEM (stock solution without supplement) | 300 µl |
| pCAGGS-NP (Z), 1 µg/µl | 0.5 µl |
| pCAGGS-P (Z)/4C(-), 1 µg/µl | 0.5 µl |
| pCAGGS-L (TDK), 1 µg/µl | 2.0 µl |
| pCAGGS-T7, 1 µg/µl | 1.0 µl |
| pMiniSeV/GFP/M/F/HN cDNA, 1 µg/µl | 5 µl |

(2) LipofectAMINE 2000 reagent [Cat No. 11668-019, Lot No. 1188609, Invitrogen] solution (per well)

| | |
|---|---|
| Opti-MEM (stock solution without supplement) | 300 µl |
| LipofectAMINE2000 | 18 µl |

(3) Five minutes after preparation of the LipofectAMINE 2000 reagent solution of (2), it was mixed with the DNA solution of (1), and left at room temperature for 20 to 30 minutes.
2.3 After washing once with Opti-MEM (stock solution), 0.6 ml of 20% FBS/Opti-MEM was added to each well, and then, 0.5 ml of the above-described transfection solution was added to each well of cells.
2.4 After incubation at 37°C for six hours, 1.2 ml of 10% FBS/G-MEM was added to each well. After washing once with VP-SFM on the next day, 2.5 µg/ml trypsin/VP-SFM was added, and the cells were cultured. Since then, the medium was exchanged every day. The supernatant (P0 virus solution) was collected from day four to day six, and production cells were infected with the virus (passage from P0 to P1 virus) for virus production. BHK-21 cells, into which pCAGGS-NP (Z), pCAGGS-P (Z)/4C (-), pCAGGS-L (TDK), and pCI-4C/P(-) were introduced on the previous day, were used as the production cells.

### 3. Construction of pMiniSeV_{SP}/GFP and pMiniSeV_{SP}/luciferase

pMiniSeV_{SP}/GFP and pMiniSeV_{SP}/luciferase, in which a promoter was linked upstream of the GFP and luciferase genes, respectively, were constructed.
The cDNA sequence of SP (single promoter):
5'-accaaacaagagaaaaaacatgtatgggatatgtaatgaagttatacaggattttagggtcaaagtatccaccctgaggagcaggttcca gaccct (96 nt) (SEQ ID NO: 20)

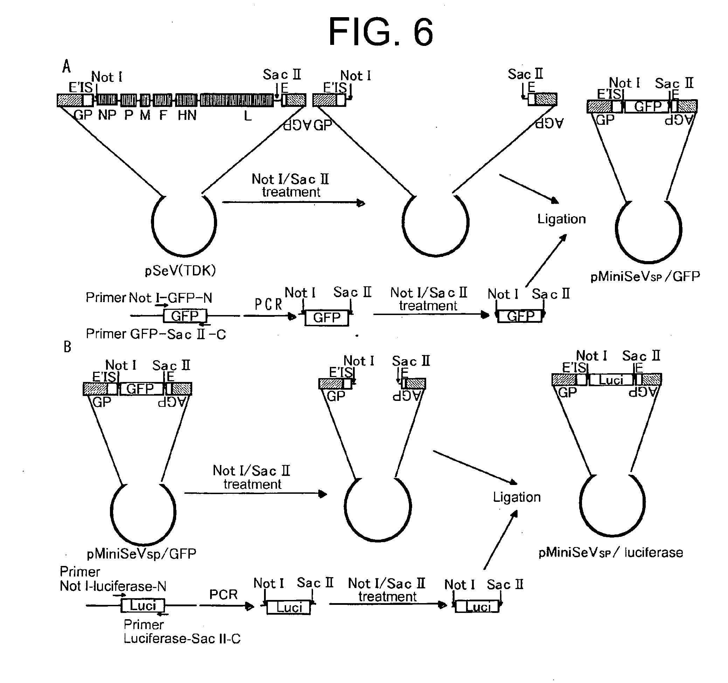

As shown in Fig. 6A, a NotI-GFP-SacII fragment was obtained by PCR using a commercially available GFP plasmid as a template and the primers NotI-GFP-N (5'-agttcacgcggccgcagatcttcacgatggtgagcaagggcgaggagctg, 50mer (SEQ ID NO: 10)) and GFP-SacII-C (5'- caggtaccgcggagcttcgatcgttctgcacgatagggactaattacttgtacagctcgtccatg, 65mer (SEQ ID NO: 11)). Then, the fragment was treated with *Not*I and *Sac*II. After purification, the fragment was inserted as a GFP insert into the pSeV(TDK) vector, which had been similarly treated with *Not*I and *Sac*II to remove a fragment containing the SeV NP, P, L, M, F, and HN sequences. Thus, pMiniSeV_{SP}/GFP (SEQ ID NO: 12) was obtained.

On the other hand, as shown in Fig. 6B, *Not*I and *Sac*II restriction sites were added to the respective ends of the luciferase gene by PCR using the primers NotI-luciferase-N (5'-atccatcgcggccgcagatcttcacgatggaagacgccaaaaacataaag, 50mer (SEQ ID NO: 13)) and Luciferase-SacII-C (5'- acttactccgcggttcgatcgttctgcacgatagggactaattacacggcgatctttccgcccttc, 66mer (SEQ ID NO: 14)). Then, pMiniSeV_{SP}/luciferase (SEQ ID NO: 15) was obtained by substituting the PCR fragment for the NotI-GFP-SacII sequence of pMiniSeV_{SP}/GFP.

### 4. Construction of pMiniSeV_{DP}/GFP and pMiniSeV_{DP}/luciferase

pMiniSeV_{DP}/GFP and pMiniSeV_{DP}/luciferase, in which two promoters were linked upstream of the GFP and luciferase gene, respectively, were constructed.
The cDNA sequence of DP (dual promoters):
accaaacaagagaaaaaacatgtatgggatatgtaatgaagttatacaggattttagtgtcaaagtaTccaccctgaggagcaggttccaga ccct / aaaaaacatgtatgggatatgtaatgaagttatacaggattttagggtcaaagtatccaccctgaggagcaggttccagaccct (GP58A (96 nt) + GPd12 (84 nt)) (SEQ ID NO: 21)

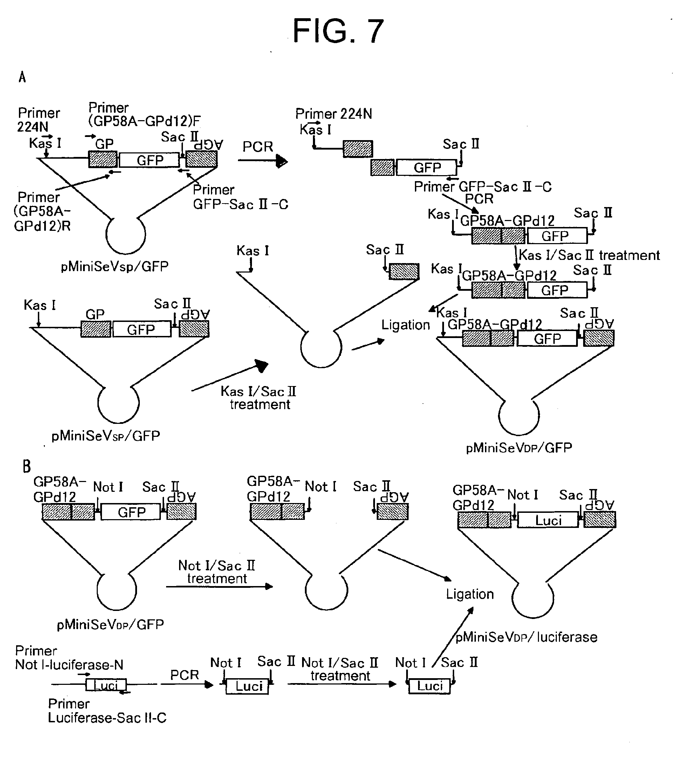

As shown in Fig. 7A, fragments were obtained by PCR using pMiniSeV_{SP}/GFP as a template, and the primers 224N (5'- ataccgcatcaggcgccattcg, 22mer (SEQ ID NO: 16)) and (GP58A-GPd12)R (5'- gttttttagggtctggaacctgctcctcagggtggatactttgacactaaaatcctg, 57mer (SEQ ID NO: 17)), or the primers (GP58A-GPd12)F (5'- ggttccagaccctaaaaaacatgtatgggatatg, 34mer (SEQ ID NO: 18)) and GFP-SacII-C (SEQ ID NO: 11). Both fragments were purified. Using these fragments as templates, a fragment was obtained by PCR using the 224N and GFP-SacII-C primers. This fragment was treated with *Kas*I and *Sac*II. After purification, the fragment was inserted into the pMiniSeV_{SP}/GFP vector, which had been similarly treated with *Kas*I and *Sac*II to remove the sequence containing the single promoter. Thus, pMiniSeV_{DP}/GFP (SEQ ID NO: 19) was obtained.

As shown in Fig. 7B, *Not*I and *Sac*II restriction sites were added to the respective ends of the luciferase gene by PCR using the primers NotI-luciferase-N and Luciferase-SacII-C. Then, pMiniSeV_{DP}/luciferase was obtained by substituting the PCR fragment for the NotI-GFP-SacII sequence of pMiniSeV_{DP}/GFP.

### 5. Reconsititution and production of MiniSeV vectors (MiniSeV_{SP}/GFP, MiniSeV_{SP}/luciferase, MiniSeV_{DP}/GFP, and MiniSeV_{DP}/luciferase)

5.1 Preparation
(1) Cells: the day before, about 8e5 BHK-21 cells/well (6-well plate)
(2) Opti-MEM [Cat No. 31985-070, Lot No. 1183337, Invitrogen] containing 20% FBS (without P&S)
(3) Opti-MEM (stock solution)

5.2 Preparation of transfection solution
(1) DNA solution (per well)

| | |
|---|---|
| Opti-MEM (stock solution without supplement) | 300 µl |
| pCAGGS-NP (Z), 1 µg/µl | 0.5 µl |
| pCAGGS-P (Z)/4C(-), 1 µg/µl | 0.5 µl |
| pCAGGS-L (TDK), 1 µg/µl | 2.0 µl |
| pCAGGS-M (Z), 1 µg/µl | 1.0 µl |
| pCAGGS-F5R, 1 µg/µl | 1.0 µl |
| pCAGGS-HN(Z), 1 µg/µl | 1.0 µl |
| pCAGGS-T7, 1 µg/µl | 1.0 µl |
| MiniSeV vector cDNA, 1 µg/µl | 5 µl |

(2) LipofectAMINE 2000 reagent [Cat No. 11668-019, Lot No. 1188609, Invitrogen] solution (per well)

| | |
|---|---|
| Opti-MEM (stock solution without supplement) | 300 µl |
| LipofectAMINE2000 | 21 µl |

(3) Five minutes after preparation of the LipofectAMINE 2000 reagent solution of (2), it was mixed with the DNA solution of (1), and left at room temperature for 20 to 30 minutes.
5.3 After washing once with Opti-MEM (stock solution), 0.6 ml of 20% FBS/Opti-MEM was added to each well, and then, 0.5 ml of the above-described transfection solution was added to each well of cells.
5.4 After culturing the above-described cells at 37°C for six hours, 1.2 ml of 10% FBS/G-MEM was added to each well. After washing once with VP-SFM on the next day, 2.5 µg/ml trypsin/VP-SFM was added, and the cells were cultured. Since then, the medium was exchanged every day. The supernatant (P0 virus solution) was collected from day three to day six, and production cells were infected with the virus (passage from P0 to P1 virus) for virus production. BHK-21 cells, into which pCAGGS-NP (Z), pCAGGS-P (Z)/4C (-), pCAGGS-L (TDK), pCI-4C/P(-), pCAGGS-M(Z), pCAGGS-F5R, and pCAGGS-HN(Z) were introduced one day before the virus infection, were used as the production cells.

### [Example 1] Effect of the SeV C protein on the productivity of MiniSeV/GFP/M/F/HN

Using the Lipofectamine 2000 reagent, pCAGGS-NP (Z), pCAGGS-P (Z)/4C(-), pCAGGS-L (TDK), and pCI control or pCI-4C/P(-) were introduced into BHK-21 cells, which were plated at 2e5 cells/well (12-well collagen-coated plate, IWAKI) on the previous day. On the next day, the cells were infected with MiniSeV/GFP/M/F/HN as a seed virus. Then, virus production was performed using VP-SFM medium containing trypsin (at a final concentration of 2.5 µg/ml). GFP fluorescence in production cells was photographed four days after infection. Furthermore, the culture supernatant was collected on day four, and fresh LLC-MK2 cells were infected with the virus (24-well plate). GFP fluorescence was photographed three days after infection.

Higher virus productivity was achieved under the conditions in which the C protein expression plasmid [pCI-4C/P(-)] was introduced, as compared to the conditions in which the control plasmid [pCI-neo], which did not express the C protein, was introduced (Fig. 3). Furthermore, it was demonstrated that expression of an appropriate level of the C protein was required for high productivity.

### [Example 2] Production of MiniSeV/GFP/M/F/HN

Using the Lipofectamine 2000 reagent, pCAGGS-NP (Z), P (Z)/4C(-), L (TDK), and pCI-4C/P(-) were introduced into BHK-21 cells, which were plated at 8e5 cells/well (6-well collagen-coated plate, IWAKI) on the previous day. On the next day, the cells were infected with MiniSeV/GFP/M/F/HN as a seed virus. Then, virus production was performed using VP-SFM medium containing trypsin (at a final concentration of 2.5 µg/ml). Every day during the period of one to seven days after infection, GFP fluorescence was photographed, the culture supernatant was collected, and the medium was exchanged. CIU assay was carried out using the culture supernatants collected from day one to day seven.

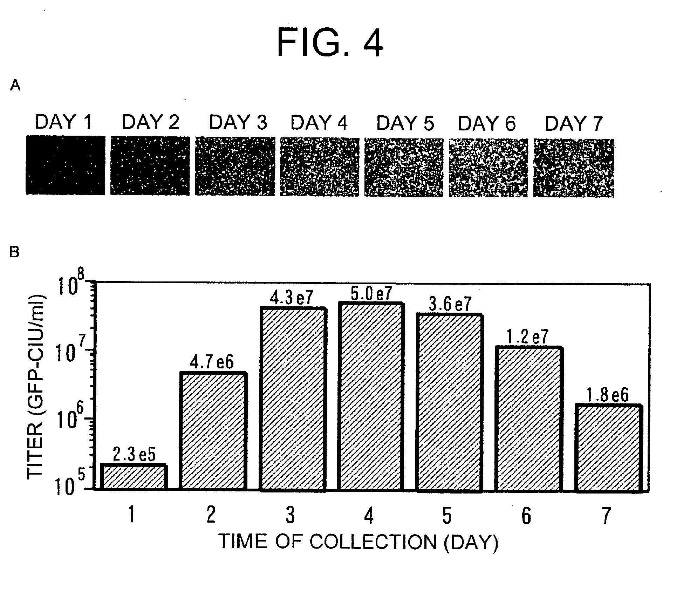

As shown in Fig. 4, MiniSeV/GFP/M/F/HN with a very high titer of 1e7 GFP-CIU/ml or more was collected from the day-3 to day-6 culture supernatants.

### [Example 3] In vitro expression using MiniSeV/GFP/M/F/HN

HeLa cells, which were plated at 3e5 cells/well (12-well collagen-coated plate, IWAKI) on the day of infection, were infected with MiniSeV/GFP/M/F/HN at an MOI of 100, 10, or 1. On the other hand, HeLa cells were co-infected with MiniSeV/GFP/M/F/HN at MOI 1 and SeVagp55/dF at MOI 5. GFP fluorescence was photographed one and two days after infection.

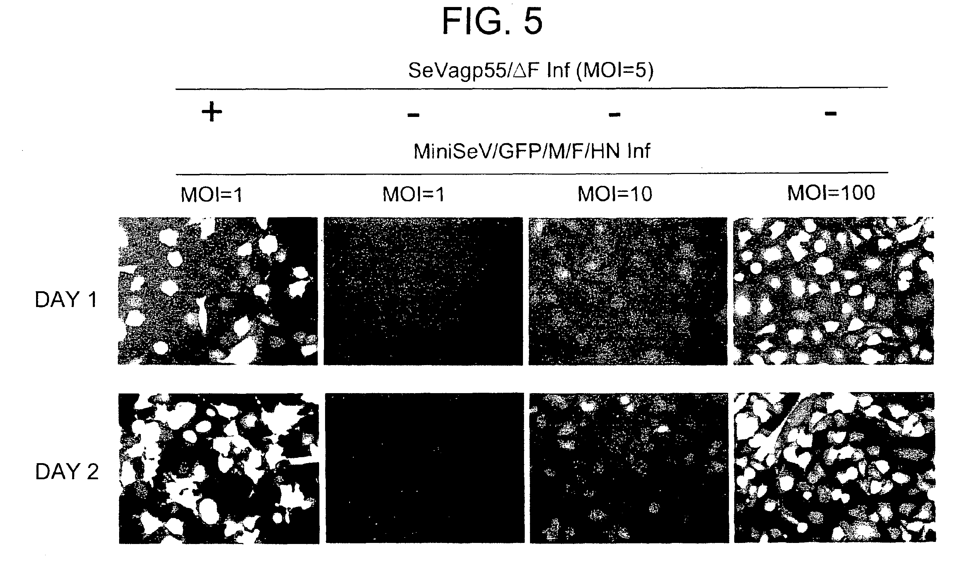

As shown in Fig. 5, only weak GFP expression was observed when the cells were infected with MiniSeV/GFP/M/F/HN alone at MOI 1; however , when the cells were infected at MOI 100, the GFP expression level was confirmed to be closer to the level when Helper SeV (SeVagp55/ΔF) was co-transfected (equivalent to a replicative SeV).

### [Example 4] Effect of genome promoters on the expression level of MiniSeV vectors

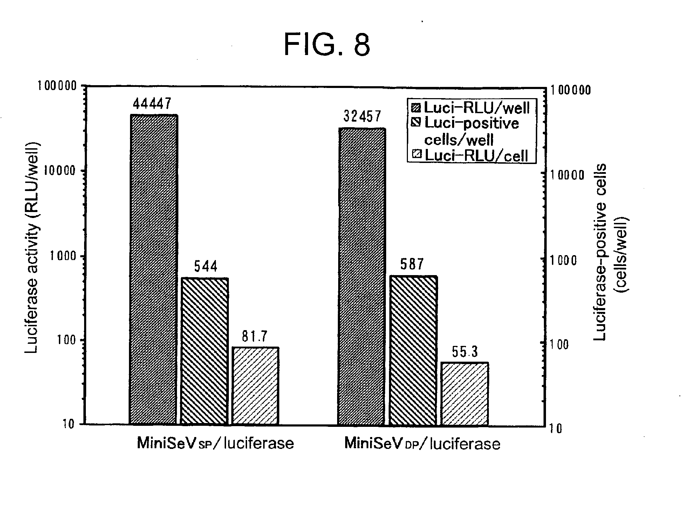

LLC-MK₂ cells, which were plated at 2e5 cells/well (12-well collagen-coated plate, IWAKI) on the previous day, were infected with either MiniSeV_{SP}/luciferase or MiniSeV_{DP}/luciferase alone (n=3), or co-infected with either of the two vectors and Helper SeV (MOI 10) (n=3). Two days after infection, cells infected with a single vector were collected from the wells to assay the luciferase activity. Cells co-infected with either of the two MiniSeV vectors and Helper SeV were immunostained in the wells using an anti-luciferase antibody to count the number of luciferase-positive cells in each well. The luciferase activity per cell can be calculated by dividing the average number of luciferase-positive cells by the average luciferase activity. As shown in Fig. 8, when the MiniSeV_{SP}/luciferase vector was used with a single promoter, the protein expression level (luciferase activity) per cell was demonstrated to be about 1.5 times that of MiniSeV_{DP}/luciferase with dual promoters.

### [Example 5] GFP expression using MiniSeV_{SP}/GFP

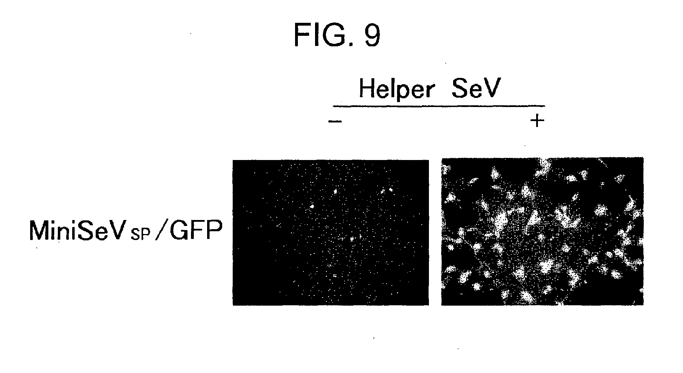

LLC-MK₂ cells, which were plated at 2e5 cells/well (12-well collagen-coated plate, IWAKI) on the previous day, were infected with MiniSeV_{SP}/GFP alone, or co-infected with MiniSeV_{SP}/GFP and Helper SeV (MOI 10). GFP fluorescence was observed and photographed two days after infection. As shown in Fig. 9, although GFP expression by infection with the MiniSeV vector alone was weaker as compared to co-infection with MiniSeV_{SP}/GFP and Helper SeV, GFP-expressing cells were clearly observed even when only the MiniSeV vector was introduced.

### Industrial Applicability

Since the non-replicating SeV vectors developed by the present inventors have high transfer efficiency and low immunogenicity, the vectors are expected to be applicable to a wide range of fields, such as antibody production, functional analysis of proteins, gene therapy, and gene vaccination.

## Claims

1. A vector that comprises a complex comprising:
(a) a negative single-stranded RNA derived from a *Paramyxoviridae* virus, wherein said negative single-stranded RNA has been modified so as not to express one or more proteins, selected from the NP, P, and L proteins, including at least the L protein, which bind to a *Paramyxoviridae* virus negative single-stranded RNA; and
(b) the NP, P, and L proteins.

2. A vector that comprises a complex comprising:
(a) a negative single-stranded RNA derived from a *Paramyxoviridae* virus, wherein said negative single-stranded RNA has been modified so as not to express multiple proteins selected from the NP, P, and L proteins, which bind to a *Paramyxoviridae* virus negative single-stranded RNA; and
(b) the NP, P, and L proteins.

3. The vector of claim 1 or 2, wherein the *Paramyxoviridae* virus-derived negative single-stranded RNA has been modified so as not to express the NP, P, and L proteins.

4. The vector of any one of claims 1 to 3, wherein the *Paramyxoviridae* virus-derived negative single-stranded RNA expresses at least one of the *Paramyxoviridae* virus envelope proteins.

5. The vector of claim 4, wherein the *Paramyxoviridae* virus-derived negative single-stranded RNA has been modified so as to express the M, F, and HN proteins, and not to express the NP, P, and L proteins.

6. The vector of any one of claims 1 to 5, wherein the negative single-stranded RNA is derived from Sendai virus.

7. The vector of any one of claims 1 to 6, wherein the negative single-stranded RNA further carries a foreign gene.

8. A DNA encoding a negative single-stranded RNA comprised in the vector of any one of claims 1 to 7, or the complementary strand thereof.

9. A method for producing the vector of claim 1, wherein the method comprises the steps of:
(a) introducing a DNA encoding a *Paramyxoviridae* virus-derived negative single-stranded RNA, or the complementary strand thereof, into cells capable of expressing the NP, P, and L proteins,
wherein said negative single-stranded RNA has been modified so as not to express one or more proteins, including at least the L protein selected from the NP, P, and L proteins, which bind to a *Paramyxoviridae* virus negative single-stranded RNA; and
(b) culturing the cells, and collecting viral particles from the culture supernatant.

10. A method for producing the vector of claim 2, wherein the method comprises the steps of:
(a) introducing a DNA encoding a *Paramyxoviridae* virus-derived negative single-stranded RNA, or the complementary strand thereof, into cells capable of expressing the NP, P, and L proteins,
wherein said negative single-stranded RNA has been modified so as not to express multiple proteins selected from the NP, P, and L proteins; and
(b) culturing the cells, and collecting viral particles from the culture supernatant.

11. The production method of claim 9 or 10, wherein the cells capable of expressing the NP, P, and L proteins can further express a protein having the function of promoting formation and release of viral particles.

12. The method of claim 11, wherein the protein having the function of promoting formation and release of viral particles is the C protein.

13. A method for producing the vector of claim 1, wherein the method comprises the steps of:
(a) introducing a vector expressing the NP protein, a vector expressing the P protein, a vector expressing the L protein, and a vector into which a DNA encoding a *Paramyxoviridae* virus-derived negative single-stranded RNA, or the complementary strand thereof, is inserted, into cells capable of expressing the vectors,
wherein said negative single-stranded RNA has been modified so as not to express one or more proteins, including at least the L protein selected from the NP, P, and L proteins, which bind to a *Paramyxoviridae* virus negative single-stranded RNA; and
(b) culturing the cells, and collecting viral particles from the culture supernatant.

14. A method for producing the vector of claim 2, wherein the method comprises the steps of:
(a) introducing a vector expressing the NP protein, a vector expressing the P protein, a vector expressing the L protein, and a vector into which a DNA encoding a *Paramyxoviridae* virus-derived negative single-stranded RNA, or the complementary strand thereof, is inserted, into cells,
wherein said negative single-stranded RNA has been modified so as not to express multiple proteins selected from the NP, P, and L proteins; and
(b) culturing the cells, and collecting viral particles from the culture supernatant.

15. A method for producing the vector of claim 1, wherein the method comprises the steps of:
(a) introducing a complex comprising a paramyxovirus-derived negative single-stranded RNA and proteins that bind to said negative single-stranded RNA, into cells expressing the NP, P, L proteins and an envelope protein,
wherein said negative single-stranded RNA has been modified so as not to express one or more proteins, including at least the L protein selected from the NP, P, and L proteins, which bind to a *Paramyxoviridae* virus negative single-stranded RNA; and
(b) culturing the cells, and collecting viral particles from the culture supernatant.

16. A method for producing the vector of claim 2, wherein the method comprises the steps of:
(a) introducing a complex comprising a paramyxovirus-derived negative single-stranded RNA and proteins that bind to said negative single-stranded RNA, into cells expressing the NP, P, L proteins and an envelop protein,
wherein said negative single-stranded RNA has been modified so as not to express multiple proteins selected from the NP, P, and L proteins, which bind to a *Paramyxoviridae* virus negative single-stranded RNA; and
(b) culturing the cells, and collecting viral particles from the culture supernatant.

17. A method for producing a vector for expressing a foreign gene in target cells, wherein the method comprises the steps of:
(a) introducing a DNA encoding a *Paramyxoviridae* virus-derived negative single-stranded RNA, or the complementary strand thereof, into cells capable of expressing the NP, P, and L proteins,
wherein said negative single-stranded RNA has been modified so as not to express one or more proteins, including at least the L protein selected from the NP, P, and L proteins, and carries a foreign gene; and
(b) culturing the cells, and collecting viral particles from the culture supernatant.

18. A method for producing a vector for expressing a foreign gene in target cells, wherein the method comprises the steps of:
(a) introducing a DNA encoding a *Paramyxoviridae* virus-derived negative single-stranded RNA, or the complementary strand thereof, into cells capable of expressing the NP, P, and L proteins,
wherein said negative single-stranded RNA has been modified so as not to express multiple proteins selected from the NP, P, and L proteins, and carries a foreign gene; and
(b) culturing the cells, and collecting viral particles from the culture supernatant.

19. A method for expressing a foreign gene in target cells, wherein the method comprises the steps of:
(a) producing the vector of claim 7; and
(b) introducing the vector of claim 7 into target cells.
